# EUROPEAN PATENT APPLICATION

(11) **EP 4 461 738 A1**
(43) Date of publication of application: **13.11.2024**
(21) Application number: 23172136.6
(22) Date of filing: 08.05.2023
(51) Int. Cl.: C07K 7/06, G01N 33/58

(54) **FURIN SENSORS AND APPLICATIONS THEREOF**

(71) Applicant: Julius-Maximilians-Universität Würzburg, 97070 Würzburg (DE)
(72) Inventor: MEINEL, Lorenz, 97082 Wuerzburg (DE); GUTMANN, Marcus Otto, 97218 Gerbrunn (DE); REINHARDT, Debora, 97956 Werbach (DE); TER MORS, Bjoern, 49076 Osnabrueck (DE); DRIEßEN, Marc, 47839 Krefeld (DE)
(74) Representative: Kraus & Lederer PartGmbB

(57) **Abstract**

The present invention relates to diagnostic furin sensors, respective furin sensitive peptides and related kits. The invention further relates to methods and medical uses involving said sensors, peptides or kits, such as methods for diagnosing cancer (particularly HNSCC), in a subject, as well as to methods for determining furin activity.

## Description

### FIELD OF THE INVENTION

The present invention relates to the field of cancer, in particular head and neck cancer, such as squamous cell carcinomas of the head and neck ("HNSCC", "head and neck squamous cell carcinoma").

### BACKGROUND

Head and neck cancer is a major global health problem. It shows a 5-year survival rate of about two-thirds, while ranking among the top 10 most prevailing cancers worldwide [1, 2]. The malignancies are mostly defined as squamous cell carcinomas of the head and neck (HNSCC), which typically arise in the oral cavity, nasal cavity, pharynx and larynx [3]. Main risk factors for development of HNSCC include smoking, alcohol consumption and human papillomavirus (HPV) infection [4]. The diagnosis of HNSCC is complex and often delayed, as most patients with HNSCC (over 60 %) are diagnosed in later stages (III or IV), resulting in poorer survival rates for the effected patients, bringing up the need for earlier detection of HNSCC [5, 6]. Common methods for diagnosis include physical examination, radiographic findings, and biopsies of the tissues of interest, resulting in a TNM-classification, separated into different categories from I to IV. The stage is based on the anatomic extent of the tumor, lymph node involvement, and metastasis ("TNM": "Tumor, lymph node, metastasis") [5, 7]. TNM-classification of the tumor dictates treatment options, involving multiple specialists, that handle a wide variety of therapy options including surgery, radiotherapy, chemotherapy, chemoradiotherapy, small molecule therapy and targeted therapies e.g., anti-EGFR therapy with Cetuximab [8, 9]. Among others, innovations in surgery have improved HNSCC therapy significantly over the last decades and continuing these trends is desirable [1, 10].

The key to reaping the benefits of the various treatments is considered the (i) early detection and (ii) broad applicability/accessibility. Hence, there is a general need in the art to improve diagnosis accordingly.

Specifically, as squamous cell carcinomas of the head and neck (HNSCC) are one of most prevailing cancers worldwide and present a massive global health burden, Therapy of the malignancies would e.g. benefit greatly if overall diagnosis was shifted to an earlier stage. Consequently, there generally is a need in the art to provide for earlier diagnosis.

Moreover, particularly also in the context of head and neck cancer, there is a need in the art for further improvements of diagnosis, such as by addressing a broader population or making testing easier for medical professionals or even at the individual level.

In general, to this end point-of-care-testing (POCT) does not only offer the possibility to approach diagnostic problems via non-invasive testing, but can also address a broader population, compared to traditional diagnostic procedures performed by medical professionals [20]. The uncomplicated application paired with the simple readout makes POCT an effective tool in different medical situations ranging from infectious diseases to cancer diagnosis [21, 22]. POCT systems can be used on the individual level or by medical professionals as bedside testing, to acquire faster diagnostic information compared to traditional laboratory-based methods.

Furin is a ubiquitous transmembrane endoprotease. It is involved in a broad range of physiological cellular functions, as well as involvement in multiple diseases, ranging from COVID-19 to various cancer types [11, 12]. Furins cleavage motif in physiological conditions is described as Arg-X-Lys/Arg-Arg 1 [13, 14]. Many pathogens, however, use this consensus sequence to exploit the extracellular protease function for maturation of their envelope proteins to enable infection of the host cell [15]. In cancer on the other hand, furins aberrant activity has been described as an important point to malignancies via proteolytic activation of e.g., hormones, receptors and growth factors [16] and elevated expression or increased activity of furin has been studied to some extent in connection with cancer [17-19].

FRET techniques (where FRET stands for "Förster resonance energy transfer", which is often also referred to "fluorescence resonance energy transfer" even though it is not restricted to the use of fluorescence) are generally well known to the skilled person - and various variants exist. In principle, the techniques involve a nonradiative transfer of energy between chromophores to e.g. detect interaction of (parts of) molecules. FRET is particularly also useful as a non-destructive method in biological systems such as living cells - for example in the form of FRET biosensors using fluorescent proteins that are compatible with the biological system and which proteins may even be expressed by such systems themselves. Generally, FRET is quite sensitive to the distance between a donor molecule and an acceptor molecule or a donor moiety and an acceptor moiety of one and the same molecule.

A variant of such techniques involves the use of a "FRET pair" including a quencher such as a quenching moiety and a fluorophore. Here, the said quencher / quenching moiety may be located in proximity to said fluorophore and may e.g. be located on the same molecule, which molecule includes a protease sensitive linker ("PSL"). Upon cleavage of the said linker, the fluorophore is easily detectable as the quenching moiety is no longer in close proximity.

### SUMMARY OF THE INVENTION

As they regarded one putative potential for improvement of HNSCC to involve diagnostic biomarkers, the present inventors considered the said aberrancy of furin in cancer a potential opportunity to utilize furin as a diagnostic marker (e.g. in HNSCC), and engaged in respective detailed studies. A successful use of furin as a diagnostic marker was considered to allow bypassing blood- or tissue-based approaches via detection of elevated furin activity in the oral cavity. In addition, it was studied whether a broader population screening for aberrant biomarker activity might be possible and of benefit in this context.

Particular further aims in context with the invention were to establish (i) an easy-to-use diagnostic system that can be used by healthcare professionals or even by the patient at home and (ii) to provide the surgeon with a system to perform rapid pre-diagnostics of the operated area directly during surgery in order to better guide surgical procedures without waiting for time-consuming diagnostic tools.

The present inventors succeeded in providing the subject-matter of the invention. The subject-matter of the invention is considered to e.g. involve the following advantages:
1.) Provision of novel diagnostic furin sensors.
2.) Provision of rapid diagnostical tool (e.g. *per operationem, post operationem* or on biopsy taking during operation outside of the body).
3.) Provision of versatile, adaptable diagnostical tool (e.g. PSL exchange).
4.) Provision of easy-to-use diagnostical tool (e.g. for health care professionals).

As a result of the above studies, the present invention provides the subject-matter as defined in the claims.

In particular, the present invention provides diagnostic furin sensors, respective furin sensitive peptides and related kits, methods and medical uses involving said sensors, peptides or kits, such as methods for diagnosing cancer (particularly HNSCC), in a subject, as well as methods for determining furin activity.

In one particular embodiment, the present invention provides FRET-based furin sensors for use in medicine, particularly in the detection of HNSCC.

Moreover, the invention particularly also relates to the subject matter defined in the following items [1] to [46]:
[1] A diagnostic sensor comprising,
   a quenching moiety R1,
   a furin-sensitive peptide Pep, and
   a signaling moiety R2,
   wherein the quenching moiety R1 suppresses the signal from the signaling moiety R2 in the said sensor.
[2] The sensor of item [1],
   A) wherein the furin-sensitive peptide Pep comprises amino acid residues having the sequence P4-P3-P2-P1-P1'-P2'-P3'-P4' (SEQ ID NO: 17), wherein
      P4 is independently selected from G and R, preferably wherein P4 is R,
      P3 is T,
      P2 is A,
      P1 is independently selected from M and G, preferably wherein P1 is M,
      P1' is independently selected from T, G and S,
      P2' is A,
      P3' is G, and
      P4' is A;
      or
   B) wherein the furin-sensitive peptide Pep comprises amino acid residues having the sequence RRARSVAS (SEQ ID NO: 1) or SSARSVAS (SEQ ID NO: 2), particularly RRARSVAS.
[3] The sensor of item [1] or [2],
   wherein the furin-sensitive peptide Pep comprises amino acid residues having the sequence P4-P3-P2-P1-P1'-P2'-P3'-P4' (SEQ ID NO: 17), wherein
   P4 is independently selected from G and R, preferably wherein P4 is R,
   P3 is T,
   P2 is A,
   P1 is independently selected from M and G, preferably wherein P1 is M,
   P1' is independently selected from T, G and S,
   P2' is A,
   P3' is G, and
   P4' is A.
[4] The sensor of any one of items [1] to [3], wherein the furin-sensitive peptide Pep comprises amino acid amino residues having a sequence selected from the group consisting of GTAMTAGA (SEQ ID NO: 3), GTAMGAGA (SEQ ID NO: 4), RTAMTAGA (SEQ ID NO: 5), RTAMGAGA (SEQ ID NO: 6), RTAMSAGA (SEQ ID NO: 7), and RTAGSAGA (SEQ ID NO: 8),
   particularly selected from the group consisting of RTAMTAGA (SEQ ID NO: 5), RTAMSAGA (SEQ ID NO: 7), and RTAGSAGA (SEQ ID NO: 8),
   especially selected from RTAMTAGA (SEQ ID NO: 5) and RTAMSAGA (SEQ ID NO: 7).
[5] The sensor of item [1] or [2], wherein the furin-sensitive peptide Pep comprises amino acid residues having the sequence RRARSVAS (SEQ ID NO: 1) or SSARSVAS (SEQ ID NO: 2), particularly RRARSVAS.
[6] The sensor of any one of items [1] to [5] wherein the sensor comprises the structure R1-Pep-R2.
[7] The sensor of any one of items [1] to [6] wherein the sensor has the structure R1-Pep-R2.
[8] The sensor of any one of items [1] to [7], wherein the quenching moiety R1 comprises a moiety selected from the group consisting of commercially available quenching moieties,
   particularly wherein R1 comprises a moiety selected from the group consisting of optionally modified 4-((4-(Dimethylamino)phenyl)azo)benzoic acid, 4-((4-((E)-(2-methoxy-5-methyl-4-((E)-(4-methyl-2 nitrophenyl) diazenyl)phenyl)diazenyl)phenyl)(methyl)amino)butanoic acid), 4-((4-((E)-(2,5-dimethoxy-4-((E)-(4-nitrophenyl)diazenyl)phenyl)diazenyl)phenyl) (methyl)amino)butanoic acid), (E)-3-((4-((3-carboxypropyl)(methyl)amino) phenyl)diazenyl)-7-(diethylamino)-5-phenylphenazin-5-ium hexafluorophosphate(V), sodium 2-((E)-(4-((3-carboxypropyl)(methyl)amino)phenyl) diazenyl)-5-((E)-(4-sulfonatophenyl)diazenyl)benzenesulfonate, 9-((E)-(2,5-dimethoxy-4-((E)-(4-nitrophenyl)diazenyl)phenyl)diazenyl)-2,3,6,7-tetrahydro-1H,5H-pyrido[3,2,1-ij]quinolin-8-ol, and sodium 3,3'-((2-((E)-2-((E)-3-((E)-2-(3-(5-carboxypentyl)-1,1-dimethyl-7-sulfonato-1,3-dihydro-2H-benzo[e]indol-2-ylidene)ethylidene)-2-chlorocyclohex-1-en-1-yl)vinyl)-3,3-dimethyl-1-(3-sulfonatopropyl)-3H-indol-1-ium-5-yl)azanediyl)bis(propane-1-sulfonate.
[9] The sensor of any one of items [1] to [8], wherein R1 comprises optionally modified 4-((4-(Dimethylamino)phenyl)azo)benzoic acid, especially wherein R1 is acetylated.
[10] The sensor of any one of items [1] to [9], wherein R1 comprises Ac-K(Dabcyl)-.
[11] The sensor of any one of items [1] to [10], wherein the fluorophore R2 comprises a moiety selected from the group consisting of small organic dyes, fluorescent proteins, and quantum dots.
[12] The sensor of any one of items [1] to [11], wherein R2 comprises 5-((2-Aminoethyl)amino)naphthalene-1 -sulfonic acid.
[13] The sensor of any one of items [1] to [12], wherein R2 comprises E(Edans).
[14] The sensor of any one of items [1] to [13], further comprising a first additional moiety L1.
[15] The sensor of any one of items [1] to [14], further comprising a second additional moiety L2.
[16] The sensor of any one of items [1] to [15], further comprising
   a first additional moiety L1,and
   a second additional moiety L2.
[17] The sensor of any one of items [14] to [16], wherein the sensor has a structure selected from the group consisting of R1-L1-Pep-L2-R2, L1-R1-Pep-L2-R2, L1-R1-Pep-R2-L2 and R1-L2-Pep-R2-L2.
[18] The sensor of any one of items [14] to [17], wherein L1 comprises a moiety selected from the group consisting of linkers, particularly small peptide linkers, natural polymers and synthetic polymers.
[19] The sensor of any one of items [15] to [18], wherein L2 comprises a moiety selected from the group consisting of linkers, particularly small peptide linkers, natural polymers and synthetic polymers.
[20] The sensor of any one of items [14] to [19], wherein L1 comprises a moiety selected from the group consisting of linkers, natural polymers and synthetic polymers, and L2 comprises a moiety selected from the group consisting of linkers, natural polymers and synthetic polymers.
[21] The sensor of any one of items [1] to [20] for use in medicine.
[22] The sensor of any one of items [1] to [21] for use in a method of diagnosing cancer.
[23] The sensor for use of item [22], wherein the method comprises determining furin activity in a subject, particularly in the oral cavity of the subject,
[24] The sensor for use of item [23], wherein an increased level of furin activity relative to a control level is indicative of the subject having cancer.
[25] The sensor for use of any one of items [21] to [24], wherein the method is a method of diagnosing a subject for cancer, and wherein the method comprises:
   a) determining a level of furin activity in the subject,
   b) optionally comparing the level of furin activity to a control level of furin activity,
   c) diagnosing whether the subject has cancer, wherein an increased level of furin activity is indicative of the subject having cancer.
[26] The sensor for use of any one of items [21] to [25], wherein the method is a method of diagnosing a subject for cancer, and wherein the method comprises:
   a) determining a level of furin activity in the subject,
   b) comparing the level of furin activity to a control level of furin activity,
   c) diagnosing whether the subject has cancer, wherein an increased level of furin activity is indicative of the subject having cancer.
[27] The sensor for use of any one of items [21] to [26], wherein the subject is a mammalian subject, particularly a human subject.
[28] The sensor for use of any one of items [21] to [27], wherein the cancer is a head and neck cancer.
[29] The sensor for use of any one of items [21] to [27], wherein the subject is a mammalian subject, particularly a human subject; and wherein the cancer is a head and neck cancer.
[30] The sensor for use of any one of items [21] to [29], wherein the cancer is HNSCC.
[31] A furin-sensitive peptide having a length of from 8 to 16 amino acids, wherein the furin-sensitive peptide Pep comprises amino acid residues having the sequence P4-P3-P2-P1-P1'-P2'-P3'-P4' (SEQ ID NO: 17), wherein
   P4 is independently selected from G and R, preferably wherein P4 is R,
   P3 is T,
   P2 is A,
   P1 is independently selected from M and G, preferably wherein P1 is M,
   P1' is independently selected from T, G and S,
   P2' is A,
   P3' is G, and
   P4' is A.
[32] The peptide of item [31], wherein the peptide comprises amino acid amino residues having a sequence selected from the group consisting of GTAMTAGA (SEQ ID NO: 3), GTAMGAGA (SEQ ID NO: 4), RTAMTAGA (SEQ ID NO: 5), RTAMGAGA (SEQ ID NO: 6), RTAMSAGA (SEQ ID NO: 7), and RTAGSAGA (SEQ ID NO: 8),
   particularly selected from the group consisting of RTAMTAGA (SEQ ID NO: 5), RTAMSAGA (SEQ ID NO: 7), and RTAGSAGA (SEQ ID NO: 8),
   especially selected from RTAMTAGA (SEQ ID NO: 5) and RTAMSAGA (SEQ ID NO: 7).
[33] A furin-sensitive peptide having a length of from 8 to 16 amino acids, wherein the furin-sensitive peptide Pep comprises amino acid residues having the sequence RRARSVAS (SEQ ID NO: 1) or SSARSVAS (SEQ ID NO: 2), particularly RRARSVAS.
[34] The furin-sensitive peptide according to any one of items [31] to [33] for use in medicine, particularly wherein the use is further defined as in any one of items [21] to [30].
[35] A diagnostic kit comprising a sensor in accordance with any one of items [1] to [20].
[36] A diagnostic kit comprising a peptide in accordance with any one of items [31] to [33].
[37] A diagnostic kit comprising a sensor in accordance with any one of items [1] to [20] and a peptide in accordance with any one of items [31] to [33].
[38] A method of determining furin activity in a sample, wherein the method comprises using a sensor in accordance with any one of items [1] to [20].
[39] A method of determining furin activity in a sample, wherein the method comprises using a peptide in accordance with any one of items [31] to [33].
[40] A method of determining furin activity in a sample, wherein the method comprises using a kit in accordance with any one of items [35] to [37].
[41] The method any one of items [38] to [40] wherein the sample has been obtained from a subject, particularly a mammalian subject, especially a human subject.
[42] The method any one of items [38] to [41] wherein the method is a method of diagnosing cancer, in particular wherein an increased level of furin activity relative to the level of a control is indicative of the subject having cancer.
[43] The method any one of items [38] to [42], wherein the sample has been obtained from a subject, and the method is a method of diagnosing cancer.
[44] The method any one of items [38] to [43], wherein the sample has been obtained from the oral cavity of a subject.
[45] The method any one of items [38] to [44], wherein the method comprises
   a) determining a level of furin activity in a sample obtained from the subject,
   b) optionally comparing the level in the said sample to a control level of furin activity,
   c) diagnosing whether the subject has cancer, wherein an increased level of furin activity is indicative of the subject having cancer.
[46] The method any one of items [38] to [45], wherein the method is further defined as in any of items [21] to [30].

### DESCRIPTION OF THE FIGURES

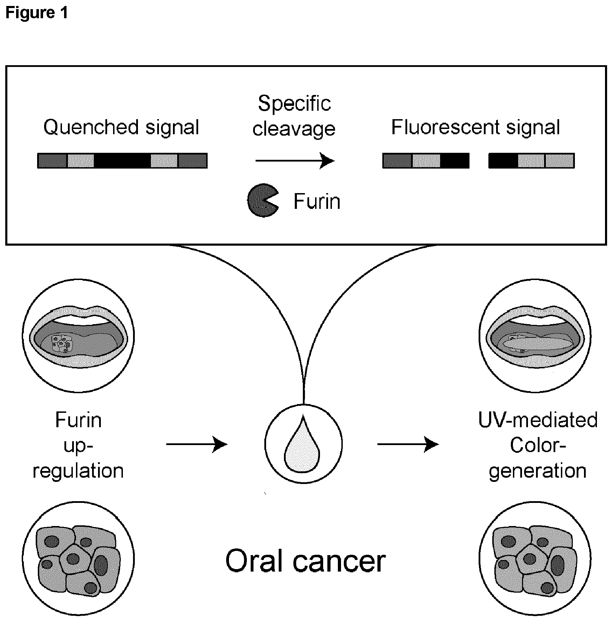

Figure 1: Schematic representation of HNSCC diagnosis using FRET-based furin sensors.

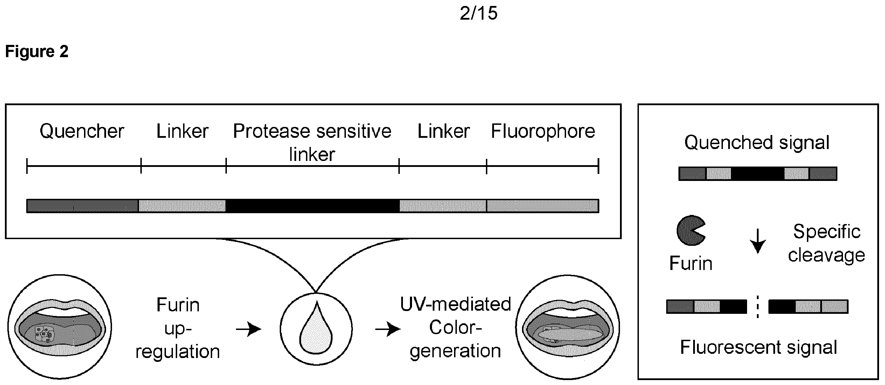

Figure 2: Overview on exemplary furin sensor. (A) Furin sensor design and principle. The sensor comprises a "FRET pair" (quencher and fluorophore), linked via a protease sensitive linker ("PSL"). Via cleavage of the PSL, the fluorophore loses the proximity of the quencher and is now susceptible for excitation.

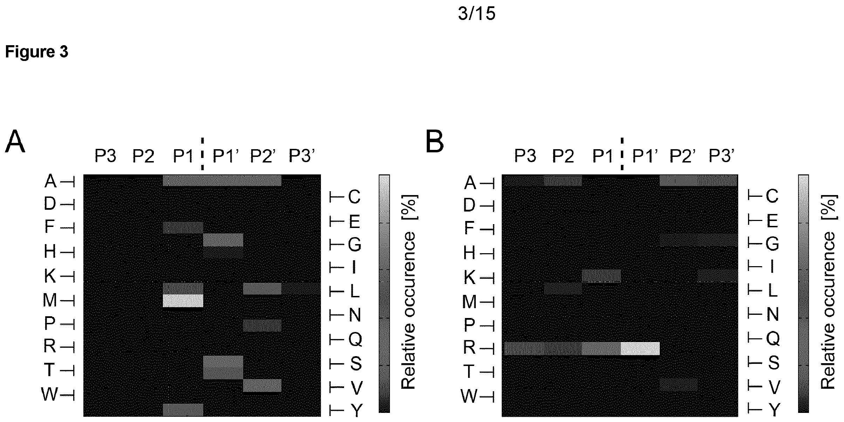

Figure 3: Furin cleavage obtained via proteomic identification of protease cleavage sites ("PICS"). (A) PICS derived relative occurrence of amino acids for position P3 to P3' acquired with trypsin-digested proteome. Aggregated results of all tryptic PICS libraries are shown (14N and 15N) with 19405 identified peptides and 3771 unique entries (B) PICS derived relative occurrence of amino acids for position P3 to P3' acquired with GluC ("Glutamyl endopeptidase GluV8")-digested proteome based on 224 identified peptides with 206 unique entries.

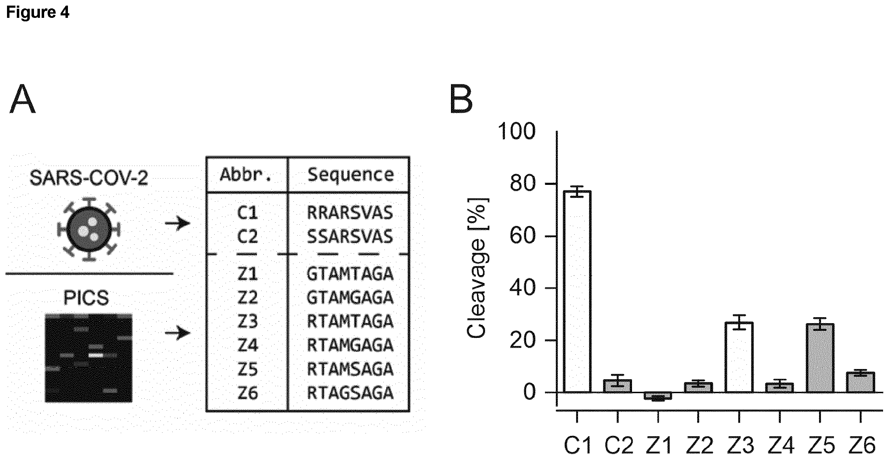

Figure 4: Protease sensitive linker identification. (A) Origin of C and Z PSLs. C-PSLs are derived from SARS-COV-2 and Z-PSLs are derived from PICS. (B) Cleavage of proposed PSLs via furin within 15 min.

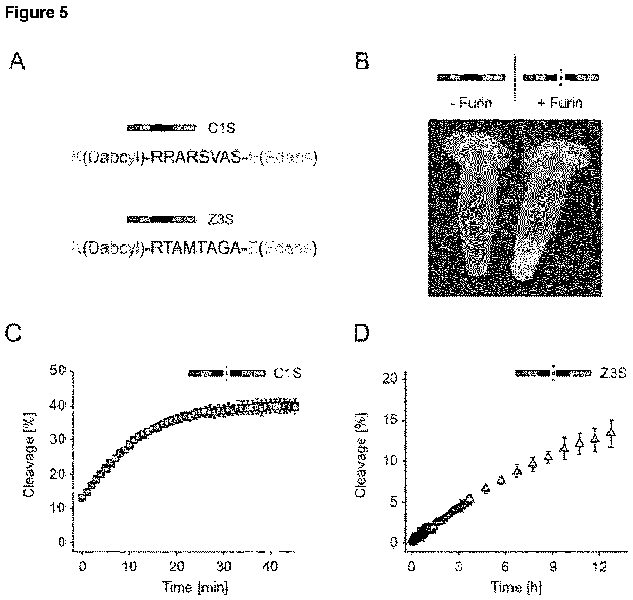

Figure 5: Characterization of "FRET-based" furin Sensors. (A) Structure of the core parts of C1S and Z3S. For sequence abbreviations the one letter amino acid code was used, except for FRET labels on the respective amino acid: 4-((4-(Dimethylamino)phenyl)azo)benzoic acid (Dabcyl) and 5-((2-Aminoethyl)amino)naphthalene-1-sulfonic acid (Edans). (B) C1S incubated without furin (left) and with furin (right) for 1 h. Illumination was performed with λ = 350 nm. (C) Cleavage of C1S over time. (D) Cleavage of Z3S over time.

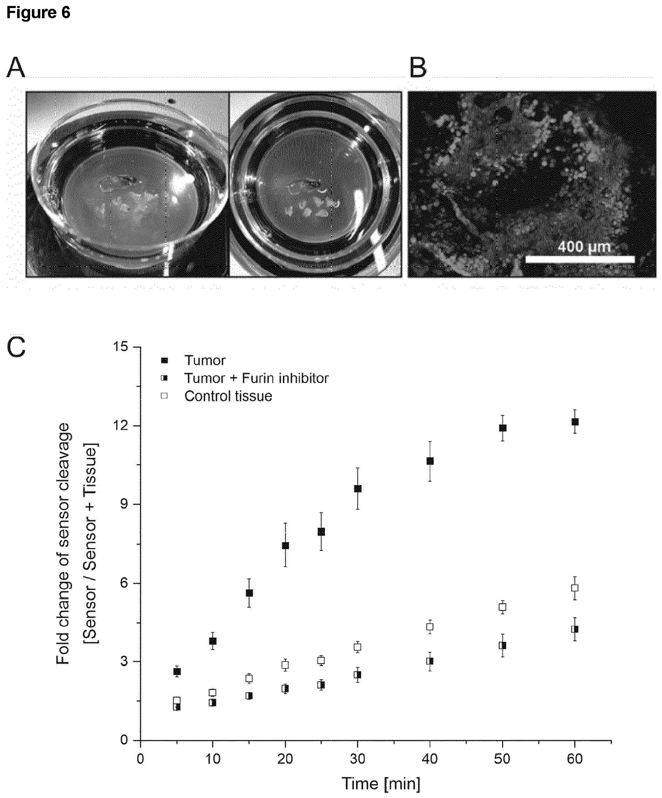

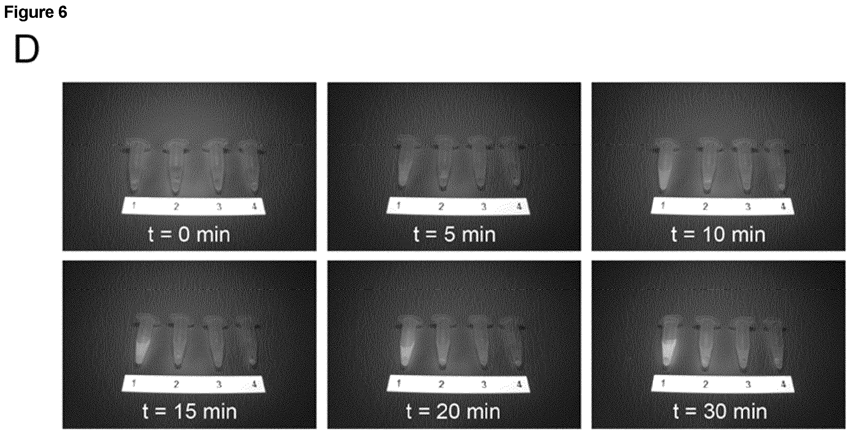

Figure 6: Characterization of HNSCC tissue. (A) HNSCC tumor tissue. (B) Tumor tissue stained with fluorescein diacetate after 24 hours of cultivation. (C) Time lapse incubation of tissues with C1S and Z3S. (1) HNSCC tissue incubated with C1S and Z3S. (2) Healthy tissue incubated with C1S. (3) HNSSC tissue incubated with Z3S. (4) Healthy tissue incubated with Z3S.

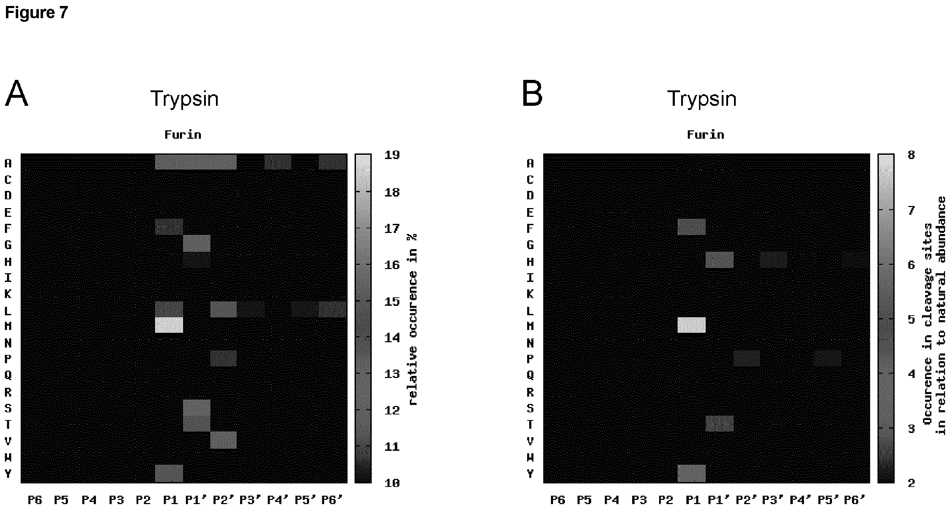

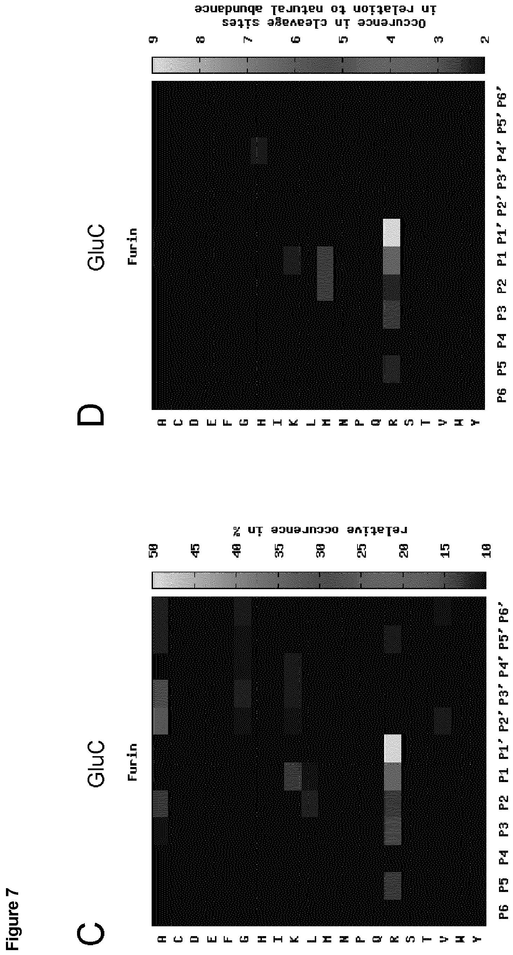

Figure 7: Furin PICS results acquired with trypsin and GluC digested proteome. Aggregated results of all tryptic pics libraries (14N and 15N) are shown with 19405 identified peptides and 3771 unique entries. For GluC 224 peptides were identified with 206 unique entries. (A) Relative occurrence of amino acids for trypsin digested proteome in %. (B) Occurrence of amino acids in cleavage sites in relation to natural abundance for trypsin digested proteome. (C) Relative occurrence of amino acids for GluC digested proteome in %. (D) Occurrence of amino acids in cleavage sites in relation to natural abundance for GluC digested proteome.

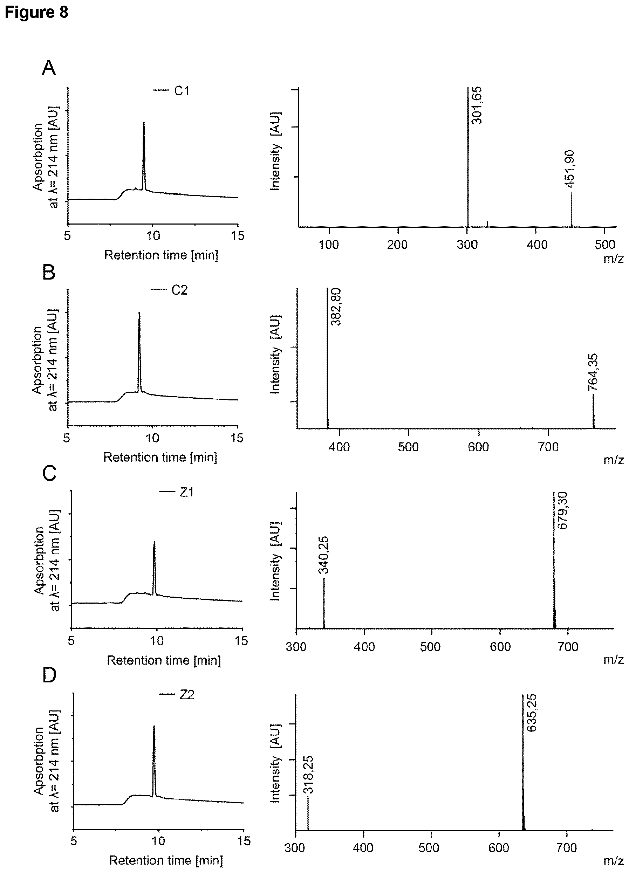

Figure 8: Characterization of C1, C2, Z1 and Z2. (A) HPLC and LC-MS analysis of C1 indicating a purity of >95% and an observed m/z of 451.90 (z = 2) for a calculated m/z of 451.75 (z =2). (B) HPLC and LC-MS analysis of C2 indicating a purity of >95% and an observed m/z of 764.35 for a calculated m/z of 764.37. (C) HPLC and LC-MS analysis of Z1 indicating a purity of >95% and an observed m/z of 679.30 for a calculated m/z of 679.29. (D) HPLC and LC-MS analysis of Z2 indicating a purity of >95% and an observed m/z of 635.25 for a calculated m/z of 635.26.

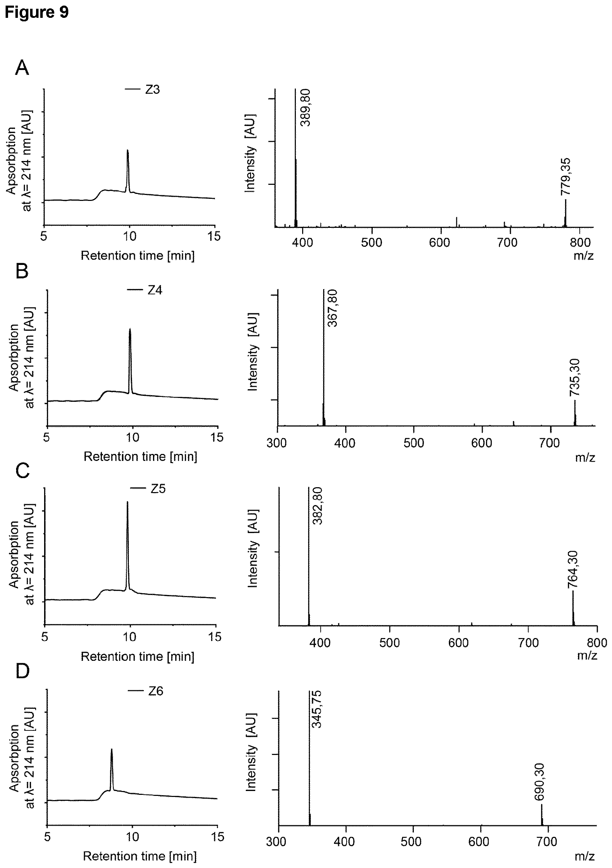

Figure 9: Characterization of Z3-6. (A) HPLC and LC-MS analysis of Z3 indicating a purity of >95% and an observed m/z of 779.35 for a calculated m/z of 778.37. (B) HPLC and LC-MS analysis of Z4 indicating a purity of >95% and an observed m/z of 735.30 for a calculated m/z of 734.34. (C) HPLC and LC-MS analysis of Z5 indicating a purity of >95% and an observed m/z of 764.30 for a calculated m/z of 764.35. (D) HPLC and LC-MS analysis of Z6 indicating a purity of >95% and an observed m/z of 690.30 for a calculated m/z of 690.34.

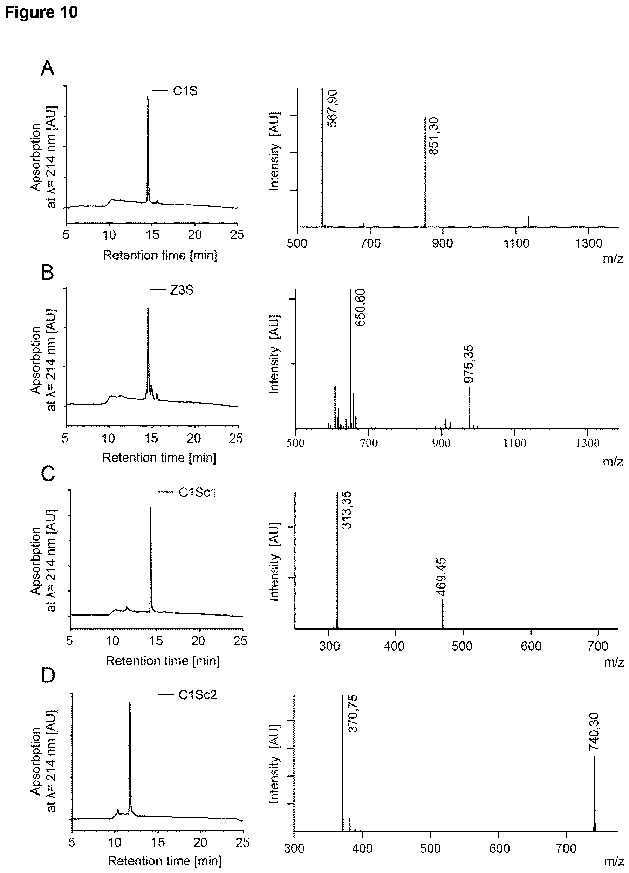

Figure 10 Characterization of C1S, Z3S, C1Sc1 and C1Sc2. (A) LC-MS analysis of C1S indicating a purity of >95% and an observed m/z of 851.30 (z =2) for a calculated m/z of 851.11 (z = 2). (B) LC-MS analysis of Z3S indicating a purity of >75% and an observed m/z of 975.35 (z =2) for a calculated m/z of 975.60 (z = 2). (C) LC-MS analysis of C1Sc1 indicating a purity of >83% and an observed m/z of 469.45 (z = 2) for a calculated m/z of 469.36 (z = 2). (D) LC-MS analysis of C1Sc2 indicating a purity of >93% and an observed m/z of 740.30 for a calculated m/z of 740.48.

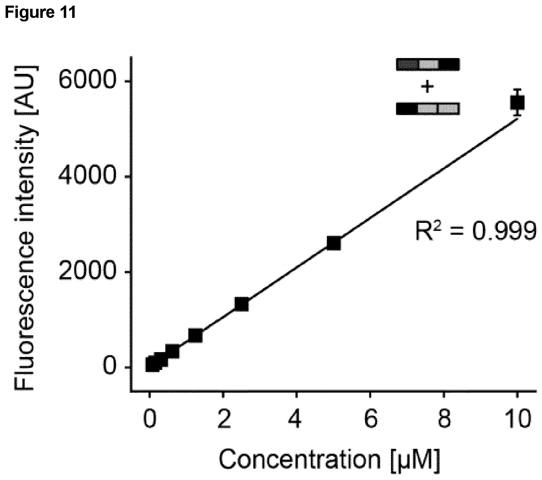

Figure 11: Concentration series of fragments C1Sc1 and C1Sc2 (1 µM represents 1 µM of each fragment).

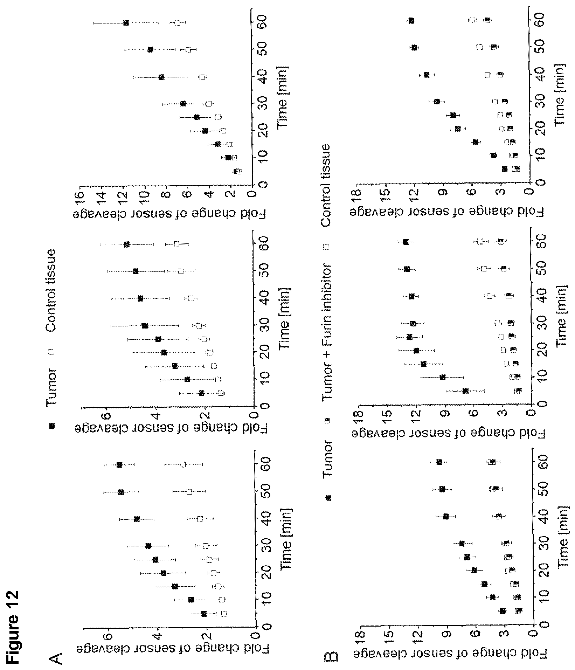

Figure 12: C1S tissue incubation. C1S incubation with tumor tissue, healthy tissue, and tumor tissue with furin inhibitor in biological replicates.

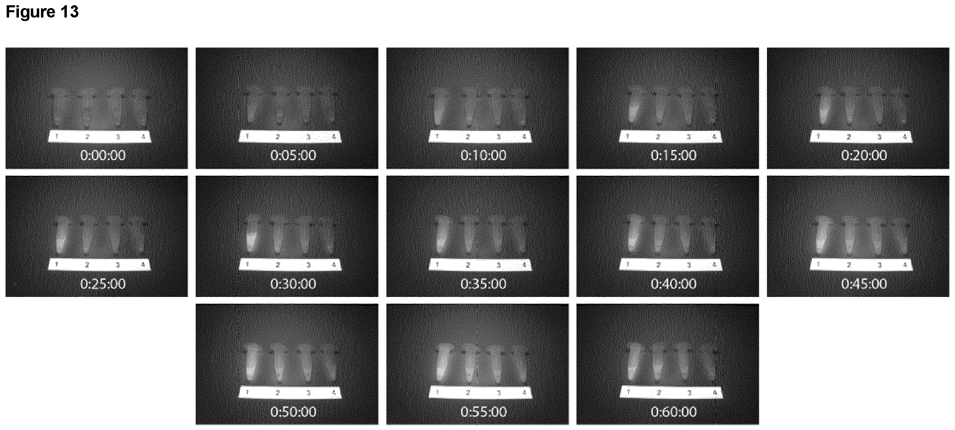

Figure 13: Time lapse incubation of tissues with sensor. (1) HNSCC tissue incubated with C1S. (2) Healthy tissue incubated with C1S. (3) HNSSC tissue incubated with Z3S. (4) Healthy tissue incubated with Z3S.

### DETAILED DESCRIPTION

In a first aspect, the present invention relates a diagnostic sensor comprising a quenching moiety R1, a furin-sensitive peptide Pep, and a signaling moiety R2.

Without intending to be bound by theory, the said quenching moiety R1 suppresses the signal from the signaling moiety R2 in the said sensor. Consequently, once the furin-sensitive peptide has been cleaved by furin, the signaling moiety is easily detectable due to spatial absence of the quenching moiety.

In a set of preferred embodiments of the first aspect, the furin-sensitive peptide Pep comprises amino acid residues having the sequence P4-P3-P2-P1-P1'-P2'-P3'-P4' (SEQ ID NO: 17), wherein P4 is independently selected from G and R, preferably wherein P4 is R, P3 is T, P2 is A, P1 is independently selected from M and G, preferably wherein P1 is M, P1' is independently selected from T, G and S, P2' is A, P3' is G, and P4' is A.

Preferably, wherein said furin-sensitive peptide Pep comprises amino acid amino residues having a sequence selected from the group consisting of GTAMTAGA (SEQ ID NO: 3), GTAMGAGA (SEQ ID NO: 4), RTAMTAGA (SEQ ID NO: 5), RTAMGAGA (SEQ ID NO: 6), RTAMSAGA (SEQ ID NO: 7), and RTAGSAGA (SEQ ID NO: 8), particularly selected from the group consisting of RTAMTAGA (SEQ ID NO: 5), RTAMSAGA (SEQ ID NO: 7), and RTAGSAGA (SEQ ID NO: 8), especially selected from RTAMTAGA (SEQ ID NO: 5) and RTAMSAGA (SEQ ID NO: 7).

In a further set of preferred embodiments of the first aspect, the furin-sensitive peptide Pep comprises amino acid residues having the sequence RRARSVAS (SEQ ID NO: 1) or SSARSVAS (SEQ ID NO: 2), particularly RRARSVAS.

In the first aspect, the sensor may comprise the structure R1-Pep-R2. Alternatively, the sensor may have the structure R1-Pep-R2.

In particular embodiments of the first aspect, the quenching moiety R1 comprises a moiety selected from the group consisting of commercially available quenching moieties, particularly selected from the group consisting of Dabcyl and the quenching moieties commercially available under the trade names Dark Fluorescent Quencher, Black Hole Quenchers, Tide Quencher, BlackBerry Quencher, Qxl quencers, Iowa Black FW, Iowa Black RQ, IRDye QC-1, Eclipse.

Preferably, Black Hole Quenchers (BHQ) are selected from the following group:
BHQ-1 (4-((4-((E)-(2-methoxy-5-methyl-4-((E)-(4-methyl-2 nitrophenyl)diazenyl)phenyl)diazenyl)phenyl)(methyl)amino)butanoic acid),
BHQ-2 (4-((4-((E)-(2,5-dimethoxy-4-((E)-(4-nitrophenyl)diazenyl)phenyl)diazenyl)phenyl)(methyl)amino)butanoic acid).
BHQ-3 ((E)-3-((4-((3-carboxypropyl)(methyl)amino)phenyl)diazenyl)-7-(diethylamino)-5-phenylphenazin-5-ium hexafluorophosphate(V), and
BHQ-10 (Sodium 2-((E)-(4-((3-carboxypropyl)(methyl)amino)phenyl)diazenyl)-5-((E)-(4-sulfonatophenyl)diazenyl)benzenesulfonate).

Preferably, the BlackBerry Quencher is BBQ-650 (9-((E)-(2,5-dimethoxy-4-((E)-(4-nitrophenyl)diazenyl)phenyl)diazenyl)-2,3,6,7-tetrahydro-1H,5H-pyrido[3,2,1-ij]quinolin-8-ol).

Besides, as will be recognized by the skilled person, IRDye QC-1 maybe also described as sodium 3,3'-((2-((E)-2-((E)-3-((E)-2-(3-(5-carboxypentyl)-1,1-dimethyl-7-sulfonato-1,3-dihydro-2H-benzo[e]indol-2-ylidene)ethylidene)-2-chlorocyclohex-1-en-1-yl)vinyl)-3,3-dimethyl-1-(3-sulfonatopropyl)-3H-indol-1-ium-5-yl)azanediyl)bis(propane-1-sulfonate.

In any case, such quenchers are well known to and readily available by the person skilled in the art and the above list is a non-limiting list of particular examples.

In specific embodiments, R1 comprises optionally modified 4-((4-(Dimethylamino)phenyl)azo)benzoic acid (also known as "Dabcyl", especially wherein R1 is acetylated. In a further particular embodiment, R1 comprises Ac-K(Dabcyl).

In particular embodiments of the first aspect, the fluorophore R2 comprises a moiety selected from the group consisting of small organic dyes, fluorescent proteins, and quantum dots.

In particular embodiments, the fluorophore R2 comprises a small organic dye, particularly selected from the group consisting of fluoresceins, rhodamines and particular commercially available small organic dyes (particularly selected from CF-Dyes, Alexa Fluor, DyLight Fluor, and Atto).

In particular embodiments, the fluorophore R2 comprises a fluorescent protein, particularly selected from GFP, CFP and particular commercially available fluorophors (such as mCherry).

In particular embodiments, the fluorophore R2 comprises a quantum dot.

Generally, suitable fluorophores are not particularly limited and will readily be selected and are readily obtainable by the skilled person.

In specific embodiments, R2 comprises 5-((2-Aminoethyl)amino)naphthalene-1-sulfonic acid (also known as "Edans"). In a further particular embodiment, R2 comprises E(Edans).

In a further set of embodiments of the first aspect, the sensor further comprises first additional moiety L1, and / or a second additional moiety L2.

In particular embodiments, the sensor comprises a structure selected from the group consisting of R1-L1-Pep-L2-R2, L1-R1-Pep-L2-R2, L1-R1-Pep-R2-L2 and R1-L2-Pep-R2-L2.

In more specific embodiments, the sensor has a structure selected from the group consisting of R1-L1-Pep-L2-R2, L1-R1-Pep-L2-R2, L1-R1-Pep-R2-L2 and R1-L2-Pep-R2-L2.

Preferably, L1 comprises a moiety selected from the group consisting of linkers, particularly small peptide linkers, natural polymers and synthetic polymers.

Preferably, L2 comprises a moiety selected from the group consisting of linkers, particularly small peptide linkers, natural polymers and synthetic polymers.

In particular embodiments, the linker is a small peptide linker, particularly GSGSGS (SEQ ID NO: 14). This is only one non-limiting example, as the skilled person will readily be in a position to select and use suitable linkers for L1 and/or L2.

In particular embodiments, the natural polymer is cellulose. This is only one non-limiting example, as the skilled person will readily be in a position to select and use suitable polymers for L1 and/or L2.

In particular embodiments, the synthetic polymer is selected from the group consisting of polyoxazoline, polyethylene glycols, polycaprolactone, and poly(vinyl)alcohols. These are only some non-limiting examples, as the skilled person will readily be in a position to select and use suitable polymers for L1 and/or L2.

Moreover, the skilled person will readily be in a position to select preferred additional moieties L1 and L2 as well as preferred pairs for L1 and L2 depending on the desired application. Such selections may e.g. be taken in view of a desired increase of hydrophilic properties, desired chemical properties, desired introduction of further functionalities and suchlike.

In a second aspect, the present invention relates to the sensor of the first aspect for use in medicine.

In a third aspect, the present invention relates to the sensor of the first aspect for use in a method of diagnosing cancer.

In preferred embodiments of the latter aspects, the use or method, respectively, comprises determining furin activity in a subject, particularly in the oral cavity of the subject.

In preferred embodiments of the second and third aspects, an increased level of furin activity relative to a control level is indicative of the subject having cancer.

In particular embodiments herein, an increased level of furin activity is indicated by a fold change of at least 5x, particularly in a time of 5-20 minutes, more preferably between 5-10 minutes, is indicative of the subject having cancer.

Especially, said increased level is determined relative to a control level established using a control without cancer.

In particular embodiments, said increased level is determined relative to a control level established using a fluorescence standard.

Depending on the particular embodiment / aspect herein, increased levels of furin activity may be determined in a subject, particularly in the oral cavity of the subject, or in a sample obtained from the subject.

In particular embodiments of the third aspect, the method is a method of diagnosing a subject for cancer, and comprises:
a) determining a level of furin activity in the subject,
b) diagnosing whether the subject has cancer, wherein an increased level of furin activity is indicative of the subject having cancer.

In more specific embodiments of the third aspect, the method comprises
a) determining a level of furin activity in the subject,
b) comparing the level of furin activity to a control level of furin activity, and
c) diagnosing whether the subject has cancer, wherein an increased level of furin activity is indicative of the subject having cancer.

In accordance with the present invention, the subject preferably is a mammalian subject, particularly a human subject.

In accordance with the present invention, the cancer preferably is a head and neck cancer. In particular embodiments, the cancer is HNSCC.

In a fourth aspect, the present invention relates to a furin-sensitive peptide having a length of from 8 to 16 amino acids, wherein the furin-sensitive peptide Pep comprises amino acid residues having the sequence P4-P3-P2-P1-P1'-P2'-P3'-P4' (SEQ ID NO: 17), wherein P4 is independently selected from G and R, preferably wherein P4 is R, P3 is T, P2 is A, P1 is independently selected from M and G, preferably wherein P1 is M, P1' is independently selected from T, G and S, P2' is A, P3' is G, and P4' is A.

In preferred embodiments, said peptide comprises amino acid amino residues having a sequence selected from the group consisting of GTAMTAGA (SEQ ID NO: 3), GTAMGAGA (SEQ ID NO: 4), RTAMTAGA (SEQ ID NO: 5), RTAMGAGA (SEQ ID NO: 6), RTAMSAGA (SEQ ID NO: 7), and RTAGSAGA (SEQ ID NO: 8).

More preferably, said peptide comprises amino acid amino residues having a sequence selected from the group consisting of RTAMTAGA (SEQ ID NO: 5), RTAMSAGA (SEQ ID NO: 7), and RTAGSAGA (SEQ ID NO: 8).

Even more preferably, said peptide comprises amino acid amino residues having a sequence selected from RTAMTAGA (SEQ ID NO: 5) and RTAMSAGA (SEQ ID NO: 7).

In a fifth aspect, the present invention relates to a furin-sensitive peptide having a length of from 8 to 16 amino acids, wherein the furin-sensitive peptide Pep comprises amino acid residues having the sequence RRARSVAS (SEQ ID NO: 1) or SSARSVAS (SEQ ID NO: 2), particularly RRARSVAS.

In a sixth aspect, the present invention relates to the peptides of the fourth and fifth aspects for use in medicine.

In preferred embodiments of the sixth aspect, the use is further defined as in any one of the second and third aspects.

In a seventh aspect, the present invention relates to a diagnostic kit comprising a sensor in accordance with the first aspect, a peptide in accordance with the fourth aspect, or a peptide in accordance with the fifth aspect.

In preferred embodiments of the seventh aspect, the sensor further defined as in the first aspect. In other preferred embodiments of the seventh aspect, the peptide is further defined as in any one of the fourth and fifth aspects.

In an eighth aspect, the present invention relates to a method of determining furin activity in a sample, wherein the method comprises using a sensor in accordance with the first aspect, a peptide in accordance with the fourth aspect, a peptide in accordance with the fifth aspect, or a kit in accordance with the seventh aspect.

In preferred embodiments, the sample has been obtained from a subject, particularly a mammalian subject, especially a human subject.

In particular embodiments, the sample has been obtained from the oral cavity of a subject.

In specific embodiments, the sample includes cells from the subject, particularly mucosal tissue.

In alternative specific embodiments, the sample includes components of cells from the subject, such as components of mucosal tissue obtained via a mucosal swap.

In a set of preferred embodiments of the eighth aspect, the method is a method of diagnosing cancer, in particular wherein an increased level of furin activity relative to the level of a control is indicative of the subject having cancer.

In particular embodiments of the eighth aspect, the method comprises
a) determining a level of furin activity in a sample obtained from the subject,
b) optionally comparing the level in the said sample to a control level of furin activity,
c) diagnosing whether the subject has cancer, wherein an increased level of furin activity is indicative of the subject having cancer.

Additional preferred embodiments of the eighth aspect correspond to those of the second and third aspects.

In the following, the studies of the present inventors underlying the present invention will be described in further detail:
The studies of the present inventors e.g. resulted in the provision of a peptide-based sensor, which comprises a furin cleavage site (protease sensitive linker - PSL), a fluorophore, and a quencher (Figure 1). Via cleavage of the sensor at the PSL, an increase in fluorescence can be observed, which indicates furin activity.

In more detail, to investigate furin as a potential a biomarker, a novel diagnostic sensor was designed. A parallel approach of Proteomic identification of protease cleavage sites (PICS) and analysis of natural sequences from SARS-COV-2 was performed to investigate possible furin cleavage sites. Cleavage sites were incorporated into a "FRET-system" as protease sensitive linkers (PSLs), "switching the sensor on", via cleavage by furin. The sensor was characterized and incubated with HNSCC tumor tissues, showing the ability to differentiate healthy from tumor tissue in less than 15 min. By highlighting aberrant furin activity, the sensor is proposed to serve as a broad screening tool for elevated furin levels, suggesting a more thorough clarification by a medical professional, or the usage as a rapid bedside method for tissue assessment during HNSCC surgery (Figure 1).

In further detail, the sensor was developed via selection of PSLs provided by proteomic identification of protease cleavage sites (PICS) and the as highly sensitive documented furin cleavage site, derived from SARS-COV-2 [12, 23]. Cleavage sites were synthesized as PSLs, characterized, and optimized for their cleavage by furin. The gained information was used to propose furin sensors. Sensors were synthesized, characterized, and tested with living tumor tissue in vitro for their furin activity.

The present inventors envisioned several possible POCT applications for the (HNSCC) diagnostic tool of the present invention. HNSCC therapy would benefit greatly, if broader populations were tested more often e.g., via a POCT test kit for home use. A second strategy could be pursued by improving HNSCC surgery due to faster tissue assessment *per operationem.* A further strategy would be using the tool of the present invention *post operationem.* Likewise, the tool of the present invention may be used outside of the body on biopsy taking during operation.

Furthermore, it was found that furin shows a secondary specificity in peptide libraries:
A novel peptide-based sensor was designed comprising a PSL linker, a quencher moiety, and a fluorophore (Figure 2). Hence, a PICS assay was performed to determine and verify the specificity of the purchased recombinant human furin. In short, the PICS assay was used to determine protease specificities by exposing a peptide library, derived from the whole proteome of an organism to the protease of choice [23, 24]. As the library was generated using a defined protease (like trypsin), analysis of newly formed cleavage sites via WebPICS allowed inferring a specificity of the studied protease. However, the outcome of this assay was strongly dependent on the protease used to create the peptide library. Hence, a PICS assay was performed in either a tryptic or GluC-digested peptide library derived from E. coli proteomes (Figure 3, Figure 7).

For GluC (Glutamyl endopeptidase GluV8) derived PICS libraries, observed results adhered to the well described specificity motif of furin, cleaving C-terminal to arginine (R-X-K/R-R|) with little preference for AA on the newly formed N-terminus. This specificity has been mapped and studied in detail [25-27]. The present inventors found R to be the primarily occurring AA for all positions from P5 up to and including P1'. This fits with long described cleavage events in e.g., Hemagglutinin (R-E-R-R|R-K-K-R) (SEQ ID NO: 13) and can also be attributed to the lack of major structural influence on determining substrate specificity (Figure 3B, Figure 7) [28]. Minor occurrences of K in P1 and A/L in P2 were observed.

Surprisingly, in tryptic libraries, furin did not only show a different specificity, but did so with good efficacy - and the present inventors identified >560 relevant and unique peptides contributing to this result. Using these libraries, the highest relative occurrences of AA observed over all positions were with methionine in position P1, with lower occurrences of alanine, tyrosine, leucine and phenylalanine (Figure 3A). In position P1' threonine showed the highest relative occurrence, next to serine, glycine and alanine. Valine, leucine, alanine and proline could be observed in position P2'. These observations resulted in a secondary specificity of X-X-X-MIS/T-X-X-X. To the best of the inventor's knowledge, this has not been described before.

Furthermore, the present inventors engaged in furin specific PSL design and optimization.

The basic furin sensor design follows the following principles: A core cleavage sequence (or PSL) of 8 AA, flanked by linkers used to attach "FRET components" and tune solubility, and finally a (FRET) donor/receptor pair at the respective termini (Figure 2).

Here, the present inventors first optimized the (core) peptide sequences to be used in the full sensor.

To define the PSL core sequence (P4-P4'), the present inventors decided to use a two-pronged approach: on the one hand a known furin target sequence of biological significance was chosen (SARS-COV-2 cleavage site S1|S2: RRAR|SVAS; C1) and a recently described mutated site (SSAR|SVAS; C2) that was shown to inhibit cell fusion of the virus, on the other information from the PICS study was used to design novel PSLs (Figure 3, Figure 4A) [12].

The latter approach was carried out stepwise, as the present inventors carefully evaluated cleavage efficiency of the novel PSLs.

Building the novel PSLs based on the interesting novel specificity of methionine in position P1, the present inventors first synthesized Z1 and Z2. The specific sequences were chosen based on three determination principles: A) AA with similar physicochemical properties were considered interchangeable (e.g. G vs A or S vs. T); B) if no significant information was available *via* PICS, the highest occurring AA was chosen (P4: G; P3: T/A, P2:A) (Figure 7); C) last the present inventors chose to avoid repeating motive AAs and filled gaps with small AA that should not have significant influence on recognition. For position P1', one example of each AA class was chosen for first tests. P2'-P4' were determined in accordance with the above principles. Following this decision process the resulting peptides, GTAMTAGA (Z1) (SEQ ID NO: 3) and GTAMGAGA (Z2) (SEQ ID NO: 4) allowed us to investigate different structural properties of AA in P1' position. C1, C2, Z1 and Z2 were synthesized, characterized (Figure 8) and tested for their sensitivity to furin within 15 minutes of incubation. C1 showed the highest observed cleavage rate of around 75 %, compared to C2 with a cleavage rate of around 5 %. For Z1 cleavage was not observed and Z2 showed a cleavage rate of around 3 %. As these rates did not correspond with the high number of cleaved peptides analyzed in PICS, the present inventors tested, whether replacing the AA in position P4 with arginine would have an impact on cleavage rates. Arginine was chosen in accordance with the known cleavage motifs [25-27]. This resulted in 4 more peptides used to test the alternative cleavage site: Z3 (RTAMTAGA) (SEQ ID NO: 5) and Z4 (RTAMGAGA) (SEQ ID NO: 6) as direct modifications of Z1 and Z2; Z5 (RTAMSAGA) (SEQ ID NO: 7) to confirm assumption of interchangeability of S/T and last Z6 (RTAGSAGA) (SEQ ID NO: 8) with structural simple amino acid glycine in P1 position to determine whether the observed cleavage at methionine is an artifact of the performed assay (Figure 9). Z3 and Z5 showed a cleavage rate of approximately 27 % and 26 % respectively, and cleavage was improved, when compared to Z1. No statistically significant difference could be observed between Z3 and Z5 (p = 0.05). Z4 showed a cleavage rate of around 3 %, comparable with Z2, thereby confirming that cleavage efficiency of the methionine-based PSLs is not only determined by P4 and P1 but also by P1' (several examples of cleavage sites can be found, where the canonical cleavage site of RXXR is followed by small aliphatic AAs in the P1' position) [27]. Finally, the present inventors determined the cleavage efficiency of Z6 to be around 8 % thereby confirming the importance of R in P4, but also demonstrating that the markedly higher cleavage of Z3 and Z6 is indeed determined by methionine in P1 position.

Finally, PSLs C1 and Z3 were chosen as cores for the furin sensors.

Next, furin sensors with different cleavage kinetics were established and tested:
Full "FRET-based" furin sensors, utilizing the well described Dabcyl/Edans system, were synthesized with PSLs C1 and Z3 (C1S, Z3S), characterized and tested for cleavage rates using human recombinant furin (Figure 5, Figure 11). To determine the cleavage rate, cleaved fragments of C1S were synthesized and a dilution series was prepared, representing the maximum theoretical fluorescent intensity of the complete cleavage of the sensor (100 %) (Figure 11). C1S as the faster performing sensor was incubated with furin to test if the change in fluorescence could be observed visually (Figure 5 B). Here, solely C1S incubated with furin showed the distinct cyan fluorescence, when illuminated at λ = 350 nm. To further evaluate cleavage rates, the fold change between Blank (Sensor without tissue) and control tissue and blank and tumor tissue was determined (Figure 12).

In the following, it was established that the furin sensors distinguish between HNSCC and healthy tissue:
HNSCC and healthy tissue samples were collected, dissected (Figure 6A) and cultured for 24 hours. To analyze cell viability, tissues sections were incubated with fluorescein diacetate (FDA) and subsequently analyzed via fluorescent microscopy (Figure 6B). Observed cells showed intense green color when analyzed. Furthermore, tissues were incubated with C1S over 60 min (Figure 6C). Cleavage was determined against blank (intact sensor) showing > 2 fold increase of the tumor tissue compared to healthy tissue. Tumor tissue treated with C1S and furin inhibitor showed similar rates compared to healthy tissue. When analyzed visually with simultaneous illumination by 365 nm LED units, C1S incubated with HNSCC tissue showed a distinct fluorescent signal after 5 min, that was amplified further over time, when compared to C1S incubated with healthy tissue (Figure 5D, Figure 13). For Z3S no difference could be observed between healthy and HNSCC tissues in the first 30 minutes. However, after 35 minutes, a significant difference was visually recognizable for Z3S.

Finally, further detailed information on peptides, sequences, residues and further results is provided in Supplementary Tables 1 to 4 located at the end of this description.

### EXAMPLES

Next, the invention will further be described by reference to the following examples. Importantly, any examples herein are intended for further illustrating the invention, but are by no means intended to limit the invention in any way.

### Example 1

### Proteomic Identification of Cleavage Sites (PICS)

Identification of protease cleavage sites was performed according to PICS described by Schilling et al. [23]. Two different PICS libraries were generated, namely E. coli ¹⁴N and E. coli ¹⁵N.

E. coli (DH5α) cells were cultured in lysogeny broth (LB) medium for 16 h at 37° C. ¹⁵N labelled cells were kept in Spectra 9 medium (Eurisotop, CGM-3030) under the same conditions. The cell suspension was centrifuged with 4000 g at 4 °C for 20 min and the supernatant was discarded. The cell pellet was washed two times via washing the pellet with PBS, following centrifugation and decantation of the supernatant. The cell pellet was kept.

Lysis of the cell pellets was carried out via suspension of the pellets in lysis buffer (125 mM Tris, 5 % SDS, 100 mM DTT, 1 mM PMSF) following three cycles of sonication (Sonopuls HD3100, Bandelin, Berlin, Germany) with 25 % amplitude, 30 s sonication, 90 s pause for 10 min on ice. After sonication the solution was adjusted to 1x radio immunoprecipitation assay (RIPA) buffer, 6 M Urea, 5% SDS and incubated for 90 min incubation at 4° C under shaking. The lysate was centrifuged at 20,000 g for 30 min, the supernatant was collected, and the concentration was determined via BCA assay.

For cysteine protection of the proteome, the lysate was adjusted to 100 mM HEPES, pH 7.5 and iodoacetamide was added to a final concentration of 20 mM. The sample was incubated for 1 h under light exclusion. After incubation, 5 mM DTT final concentration was added to quench excess iodoacetamide. The samples were adjusted to 15 % TCA (v/v) and incubated on ice for 1 h. The samples were centrifuged at 8,500 g for 1 hour at 4 °C, the supernatant was discarded, the pellets were washed twice with cold methanol and the pellet was air-dried. The pellets were solubilized in ice-cold 20 mM NaOH via ultrasonic bath for 1 h followed by sonication (Sonopuls HD3100, Bandelin, Berlin, Germany) with 25 % amplitude, 30 s sonication, 90 s pause for 10 min on ice to reach a 2 mg/mL concentration. The samples were adjusted to 200 mM HEPES, pH 7.5, centrifuged for 30 min at 8,500 g and the supernatant was kept. The concentration of the supernatant was determined via BCA assay.

To perform endoprotease digestion, proteomes were adjusted to pH 7-9 with NaOH. Following digestion with trypsin or GluC with a protease to proteome ratio of 1/100 (w/w) for 16 h at 37 °C, respectively. Digestion was verified via SDS gel, with no major bands > 10 kDa. After protease digest, the protease was inactivated by incubation at 70 °C for 20 min. The sample was cooled down and 1 mM PMSF final concentration was added. Afterwards 1 M guanidine hydrochloride final concentration was added, and the sample was centrifuged at 4500 rpm, at 4 °C for 1 h and the supernatant was kept. 5 mM DTT final concentration was added to the sample and incubated for 1 h at 37 °C.

For a second round of cysteine protection 40 mM iodoacetamide final concentration was added to the sample and incubated for 1.5 h at 37° under light exclusion. Another 15 mM DTT final concentration was added to quench excess iodoacetamide and incubated at 37 °C for 10 min.

To protect amines 30 mM formaldehyde and 30 mM sodium cyanoborohydride (final concentrations) were added and incubated at 25 °C for 2 h. Another 30 mM formaldehyde and 30 mM sodium cyanoborohydride (final concentrations) were added and incubated at 25 °C for 16 h. 100 mM glycine final concentration was added and incubated at 25 °C for 30 min. The sample was acidified with TFA to 0.5% and degassed. The samples were purified by C18 solid phase extraction and lyophilized. The lyophilized sample was resolved in 200 mM HEPES, pH 7.5 and the concentration was determined via BCA assay. 2 mg/ml aliquots were prepared and stored at -80 °C until further usage.

For digestion with furin, 300 µg of peptide libraries were thawed and adjusted to 100 mM HEPES pH 7.5, 5 mM CaCl₂. The peptide libraries were incubated with 5 U furin each, for 16 h at 37 °C.

To purify cleaved peptide fragments the sample was adjusted to pH 7-8, 0.5 mM sulfo-NHS-SS-biotin final concentration was added and incubated for 2 h at 25 °C. High-capacity streptavidin sepharose was equilibrated via repeated centrifugation and addition of equilibration buffer (50 mM HEPES, pH 7.5, 150 mM NaCl). The equilibrated streptavidin sepharose was added in 1.5/1 (v/v) to the biotinylated sample and incubated for 0.5 h at 22 °C. The slurry was transferred to a spin column and centrifuged. The flow-through was applied one more time to the resin and centrifuged again. The resin was washed nine times with wash buffer (50 mM HEPES, pH 7.5, 150 mM NaCl). After washing, 500 µL elution buffer (50 mM HEPES, 10 mM DTT, pH 7.5) was added to the slurry and incubated for 2 h at 25 °C. The column was centrifuged and the flow-through was applied to the resin one more time. The column was centrifuged again, and the flow-troughs were combined. Afterwards the samples were purified via solid phase extraction (StrataX, Phenomenex) (Washed with 4-5mL of 0.4%FA, elution in 1.8mL 0.4FA/80%ACN) and stored at -80 °C until further usage.

### LC-MS / Data Analysis

Samples were resuspended in loading buffer (0.2% FA/2% CAN/98%H2O) prior to analysis. NanoLC-MS/MS analyses were performed on a LTQ-Orbitrap Velos Pro (Thermo Scientific) equipped with a PicoView Ion Source (New Objective) and coupled to an EASY-nLC 1000 (Thermo Scientific). Peptides were loaded on capillary columns (PicoFrit, 30 cm x 150 µm ID, New Objective) self-packed with ReproSil-Pur 120 C18-AQ, 1.9 µm (Dr. Maisch) and separated with a 120-minute linear gradient from 3% to 30% acetonitrile and 0.1% formic acid and a flow rate of 500 nl/min.

MS scans were acquired in the Orbitrap analyzer with a resolution of 30000 at m/z 400, MS/MS scans were acquired in the Orbitrap analyzer with a resolution of 7500 at m/z 400 using HCD fragmentation with 30% normalized collision energy. A TOP5 data-dependent MS/MS method was used; dynamic exclusion was applied with a repeat count of 1 and an; singly charged precursors were excluded from selection. Minimum signal threshold for precursor selection was set to 50000. Predictive AGC was used with AGC target a value of 1e6 for MS scans and 5e4 for MS/MS scans. Lock mass option was applied for internal calibration in all runs using background ions from protonated decamethylcyclopentasiloxane (m/z 371.10124).

### Data analysis

Data analysis was performed using PMi-Byos software by Protein Metrics Inc. (United States). Data files were searched, with the following settings: Protease set as trypsin (C-terminal to R, K), semi-specific digest (ragged peptide N-terminus), up to 3 missed cleavages. Precursor Tolerance of 7.5 ppm, fragment mass tolerance of 15 ppm. Carbamidomethyl @ C as fixed modification, dimethyl/methyl/thioacyl @ K as common and thiacyl @ peptide N-term as common (residue from the cleavable enrichment probe). With max allowed common modifications at 3 and rare max set to 1. For 15N samples a fixed modification for each amino acid was used to enable search of heavy labelled peptides (see list below).

(The present inventors tested to add modifications for potentially unlabeled, or not fully labelled amino acids (e.g. 15N-remove_Gly / -0,997035 @ G), however due to the needed number of additional modifications - at least 21 and up to 30 to account for partial labeling - the present inventors found no improvement in the results generated - Data not shown).

Data was searched against the following databases:
HEK: Human TREMBL+isoforms (downloaded from uniport)
EColi: Ecoli Reference proteome (downloaded from uniport)

Search Results were filtered on peptide level to not contain reverse proteins (from decoys) and only contain peptides with a PEP 2D score of <0.1, total score >100 and containing 'N-terminal thioacyl.

The resulting peptide list was submitted to the WEB-PICs application (http://clipserve.clip.ubc.ca/pics) and the results were used to enable design of custom peptides [24].
15N - custom modifications
15N_Gly / +0.997035 @ G | fixed
15N_Ala / +0.997035 @ A | fixed
15N_Ser / +0.997035 @ S | fixed
15N_Thr / +0.997035 @ T | fixed
15N_Cys / +0.997035 @ C | fixed
15N_Val / +0.997035 @ V | fixed
15N_Leu / +0.997035 @ L | fixed
15N_Ile / +0.997035 @ I | fixed
15N_Met / +0.997035 @ M | fixed
15N_Pro / +0.997035 @ P | fixed
15N_Phe / +0.997035 @ F | fixed
15N_Tyr / +0.997035 @ Y | fixed
15N_Asp / +0.997035 @ D | fixed
15N_Glu / +0.997035 @ E | fixed
15N_Trp / + 1.994070 @ W | fixed
15N_Asn / +1.994070 @ N | fixed
15N_Gln / +1.994070 @ Q | fixed

### Example 2

### Peptide synthesis

All peptides described in this manuscript were synthesized in-house using Fmoc based solid phase peptide synthesis (SPPS).

100 µmol loaded 2-chlorotrityl chloride resin (CTC-Resin) was loaded into a polypropylene-reactor (MultiSynTech, Witten, Germany) and the first amino acid was coupled with a 5-molar excess of the respective first Fmoc-protected aminoacid. 2 mL DCM and 285 µL DIPEA were added and the mixture was incubated at 25 °C under rigorous shaking for at least 3 hours. To block free CTC-binding sites 100 µL methanol was added to the mixture and incubated again for 30 minutes at 25 °C under rigorous shaking. The resin was washed 3 times with DCM and 3 times with DMF.

The subsequent amino acids were either coupled via semi-automatic Biotage^{®} Initiator+ SP Wave peptide synthesizer or Liberty Blue^{™} automatic peptide synthesizer. Coupling via Biotage^{®} Initiator+ SP Wave peptide synthesizer was performed via solubilization of 500 µmol of the respective Fmoc-protected amino acid in 2 mL DMF containing 0.5 M hydroxybenzotriazole (HOBT), 0.5 M N,N'-diisopropylcarbodiimide (DIC) and 0.2 M DIPEA at 50 °C for 10 min, followed by three wash steps with DMF. Fmoc deprotection was carried out via incubation in 20 % piperidine in DMF for 3 min at 75 °C followed by four wash steps with DMF. Coupling of subsequent amino acids via Liberty Blue^{™} was performed with 0.2 M of the respective amino acid, in DMF containing 1 M ethyl cyanohydroxyiminoacetate (oxyma) and 0.5 M N,N'-diisopropylcarbodiimide (DIC) for 15 s at 75 °C, followed by 110 s at 90 °C. Fmoc deprotection was carried out in DMF with 20 % piperidine at 75 °C for 15 s followed by 50 s at 90 °C.

After coupling of the last amino acid, the peptide was washed three times with DCM and three times with diethylether and then dried. Cleavage of the peptide was performed via incubation in cleavage solution (trifluoroacetic acid, 2.5 % water, 2.5 % triisopropylsilane, 2.5 % 2,2'-(ethylendioxy)diethanthiol (DODT)) for 1 h at room temperature under rigorous shaking. The peptide was precipitated in ice cold diethyl ether, centrifuged, decanted and the procedure was repeated two more times.

To acetylate the N-terminus of the peptides, 2 mL of DMF was added to resin-bound peptides. Additionally, 90 µL DIPEA and 50 µL acetic anhydrite were added, following incubation of the mixture for 30 min at 25 °C under rigorous shaking. The resin was washed 3 times with DMF, 3 times with DCM and 3 times with diethyl ether, dried and stored at 4°C until further usage.

Preparative peptide purification was carried out via fast protein liquid chromatography (FPLC) on a GE ÄKTA pure (GE Healthcare, Chalfont St Giles, UK) system with a Luna 15 µm C18 100 Å column, 21.2 mm internal diameter, 250 mm length (Phenomenex Inc., Torrance, CA), with eluant A (0.1% TFA in water (v/v)) and eluent B (0.1% TFA in ACN (v/v)) with varying gradients starting from 10-50% (depending on the peptides solubility) to 100 % B in 60 minutes. UV absorption was read at λ = 214 nm 254 nm and 280 nm. The samples were frozen in liquid nitrogen and freeze-dried via CoolSafe 110-4 Pro (LaboGene A/S, Allerod, Denmark) and stored at -80 °C until further usage.

Trypsin derived peptides libraries were tested with n = 6 and GluC derived peptide libraries were tested with n = 1.

### Peptide cleavage

Lyophilized peptides were solubilized in furin buffer (100 mM HEPES pH 7.5, 5 mM CaCl₂) to 1 mM stock solutions. Cleavage was performed at 37 °C under rigorous shaking in 30 µL furin buffer containing 0.1 mM of the respective peptide and 1 U furin per reaction. The reaction was stopped via addition of 5 mM EDTA final concentration. Experiments were performed n = 3.

Analytical HPLC was carried out via an Agilent 1260 infinity II HPLC (Agilent Technologies Inc., Waldbronn, Germany) using a Zorbax 300SB-CN 5µm analytical 4.6 x 150 mm LC column (Agilent Technologies Inc., Waldbronn, Germany) The device was equipped with a diode array detector (G7115A, Agilent), a fluorescence detector (G7121A, Agilent), an automatic vial sampler (G7129C, Agilent), flexible Pump (G7104C, Agilent), and multicolumn oven (G7116A, Agilent). Mobile phase A was 0.1% TFA in Millipore water. Mobile phase B was ACN with 0.1% TFA, flow was set to 1 ml/min, injection volume was 15 µl, and the wavelength of the detector was set to λ = 214 nm.

The gradient was held at 5% B for 1 minute, then increasing to 30% within 7 minutes, increased again for 0.5 minutes to 95% B, held for 0.5 min, then back to 5% B within 0.5 minute, and held for 5.5 minutes.

Cleavage percent was analyzed by comparing the area under the curve (AUC) of the main peak from the respective peptide incubated with furin, compared to a negative control without furin.

### Example 3

### Liquid chromatography-mass spectrometry (LC-MS)

LC-MS spectra were acquired via a Shimadzu LC-MS system (Shimadzu Scientific Instruments, Columbia, MD, USA) equipped with a DGU-20A3R degassing unit, a LC20AB liquid chromatograph using a Synergi 4 µm fusion-RP column (150 x 4.6 mm) (Phenomenex Inc., Torrance, CA), SPD-20A UV/Vis detector, coupled with a single quadrupole LCMS-2020. Mobile phase A was 0.1 % (v/v) formic acid (FA) in Millipore water. Mobile phase B was methanol with 0.1 % (v/v) FA, flow was set to 1 ml/min, the wavelength of the detector was set to λ = 214 nm and the injected volume of the sample was 20 µL. The gradient was increased in 8 min from 5 to 90 % B, then held at 90 % B for 5 min, reduced to 5 % B within 1 min, and held at 5 % B for 4 min. The detection range was set to 60 to 1000 (m/z) and the spectra were measured at a voltage of 3.5 kV and a capillary temperature of 210 °C using N2 as carrier gas.

### High performance liquid chromatography (HPLC)

Analytical HPLC was carried out via an Agilent 1260 infinity II HPLC (Agilent Technologies Inc., Waldbronn, Germany) using a Zorbax 300SB-CN 5µm analytical 4.6 x 150 mm LC column (Agilent Technologies Inc., Waldbronn, Germany) The device was equipped with a diode array detector (G7115A, Agilent), a fluorescence detector (G7121A, Agilent), an automatic vial sampler (G7129C, Agilent), flexible Pump (G7104C, Agilent), and multicolumn oven (G7116A, Agilent). Mobile phase A was 0.1% TFA in Millipore water. Mobile phase B was ACN with 0.1% TFA, flow was set to 1 ml/min, injection volume was 15 µl, and the wavelength of the detector was set to λ = 214 nm for PICS derived peptides.

The gradient was held at 15% B for 1 minute, then increasing to 40% within 7 minutes, increased again for 0.5 minutes to 95% B, held for 0.5 min, then back to 15% B within 0.5 minute, and held for 5.5 minutes.

### Example 4

### Preparation of standard curve

A concentration range of mixed fragments C1Sc1 and C1Sc2 was prepared in concentrations ranging from 10- 0.078 µM of each fragment with n = 3. The samples were analyzed using an Infinite M plex (Tecan trading AG, Männedorf, Switzerland) with an excitation wavelength of λ = 340 nm and an emission wavelength of λ = 490 nm. Cleavage rates in all following experiments were calculated using the fluorescence intensity of the respective experiment and a linear equation resulting from the concentration range.

### Cleavage of FRET sensors

Sensors C1S and Z3S were incubated with 0.0333 U/µL furin in 50 µL furin buffer (100 mM HEPES pH 7.5, 5 mM CaCl₂) in concentrations of 10 µM over 12 h at 37 °C. Negative controls were performed without furin. Experiments were performed n = 3. The samples were analyzed using an Infinite M plex (Tecan trading AG, Männedorf, Switzerland) with an excitation wavelength of λ = 340 nm and an emission wavelength of λ = 490 nm.

### Illumination of C1S

Sensor C1S was incubated as described before under cleavage of FRET sensors with and without furin for 60 min and illuminated via Universal UV Lampe (CAMAG, Germany) with λ = 350 nm.

### Example 5

### Tissue collection

All included patients were diagnosed with primary oral squamous cell carcinoma (OSCC). Patients suffering from other tumor entities as well as inoperable cases were excluded from this study.

After performing preoperative tumor staging and incisional biopsy to confirm the diagnosis, stage-appropriate tumor resection, lymph-node dissection, and reconstruction took place. Tumor resection was performed in the usual way, which included using separate margins for frozen section analysis in order to confirm in sano resection. In addition to this tumor resection, a small part of corresponding oral mucosa of about 5mm (clinically non-suspicious and at a sufficient distance from the primary tumor resection to ensure safety) was excised separately. Both the resected tumor tissue and the resected healthy oral mucosa were placed in cell culture medium (RPMI-1640, Dutch modification, 10% FCS, 1% Penicillin/Streptomycin, Nystatin) and immediately transported in cooler to the Institute of Pathology for further processing. Further surgical treatment was performed without changes in the typical clinical routine.

The tissue arrived at the Institute of Pathology within one hour of surgical removal. Both specimens (the tumor and the healthy mucosa) were separately removed from their tubes and macroscopically inspected.

The healthy mucosal tissue was cut into two equal pieces, each consisting of mucosal and sparse submucosal tissue. One piece was transferred in formalin for usual fixation and subsequent histopathological assessment, while the other piece was returned to the medium and stored on ice for further investigation within the study.

The tumor specimen was orientated, measured, and then sectioned as usual using mostly small transverse sections along a plane that best demonstrated the tumor's relationship to adjacent structures. The sections were not cut through completely and still had a connection to deeper parts of the tissue in order to enable better processing (as described below). The cut surfaces were then inspected, and macroscopically visible tumor tissue was gathered from a central location in the specimen. This procedure was selected so that the removal would not interfere with the subsequent normal histopathological processing in the routine diagnostic in terms of the dissection margins as well as the tumor stage. The collected tumor tissue was then put into another tube containing the medium and was also put back on ice for the study. Afterwards, a macroscopically assumed edge area of the tumor was taken and put in a separate tube with medium and was put back on ice for the study. The cooler was then quickly passed on for further processing.

The rest of the tumor specimen was returned in its original container and fixated in formalin overnight for normal processing and histopathological assessment the next day. The areas of the specimen where the tumor and tumor edge tissue had been collected were marked. The next day, the remaining healthy mucosal specimen was processed and sectioned as usual, paraffin-embedded, and stained with hematoxylin and eosin. The tumor specimen was also processed and sectioned as usual, but adjacent tissue from the marked area where the tissue for the study had been taken was embedded separately in its own tissue cassette in order to histologically ensure that tumor tissue and tumor edge tissue had been taken from the correct part of the specimen.

### Tissue preparation

Tissues were stored on ice in sterile filtered in cell culture medium (RPMI-1640, Dutch modification, 10% FCS, 1% Penicillin/Streptomycin, Nystatin). The tissue was cut to pieces of 6.69 mg ± 2.37 mg (no statistical difference between groups could be observed with one-way ANOVA and post-hoc Tukey-test, p = 0.05) and cultivated in 16-well plates in cell culture medium at 37 °C, 5% CO₂ for 24 h. Fluorescein diacetate (FDA) stock solution was prepared by dissolving 5 mg FDA in acetone and stored at 4°C in brown glass bottles. After the 24h incubation, one of the tumor tissues was analyzed using FDA. Working solution of FDA (20 µg/mL in PBS) was freshly prepared from the FDA Stock (5 mg/mL) and 500 µL of FDA working solution was added to the tumor tissue. The tumor tissue was incubated for 5 min and analyzed via Axio Observer.Z1 microscope equipped with an A-Plan 10x/0.25 Ph1 objective (Zeiss), 38 HE Green Fluorescent Protein (λex= 450-490 nm, λem= 500-550 nm) and a mercury vapor short-arc lamp.

### FRET-based sensor tissue incubation

Tissues were prepared as described before. For incubation with FRET-Sensors, tissues were washed with reaction buffer (100 mM HEPES pH 7.5, 1 mM CaCl₂) and incubated in 200 µL reaction buffer containing 10 µM C1S, with addition of 100 µM furin inhibitor I (Merck Millipore, Darmstadt, Germany) for inhibitor controls. The tissues were incubated at 37 °C and analyzed at t = 0, 5, 10, 15, 20, 25, 30, 40, 50 and 60 min via an Infinite M plex (Tecan trading AG, Männedorf, Switzerland) with an excitation wavelength of λ = 340 nm and an emission wavelength of λ = 490 nm. Experiments were performed at least n = 3.

### Statistical analysis

Comparisons between groups were performed by one-way ANOVA using OriginPro v.2021 with post-hoc Tukey Test. P-values >0.5 were considered statistically significant.

### Time Lapse tissue incubation

22 mg of tissue (HNSCC tissue and healthy tissue) were incubated for 1 h at 37 °C with 500 µL (10 µM) of C1S or Z3S, respectively. Illumination was performed by a LED unit (16x60 mm) with three 3x Luminus SST-10 UV 365nm-370nm on a SYSTEM Slider (Led-tech.com) controlled by SLT6 - 350 IFG (self-electronics) control unit using 365 nm and 350 mA as control settings.

**Supplementary Table 1. Overview of peptides and sensors detailed in this description. Sequence Abbreviations: The one letter amino acid code was used, except for N-terminal acetylation (Ac), and for FRET labels on the respective amino acid: 4-((4-(Dimethylamino)phenyl)azo)benzoic acid (Dabcyl) and 5-((2-Aminoethyl)amino)naphthalene-1-sulfonic acid (Edans)**

| Abbreviation | SEQ ID NO: | Sequence | Characterization |
|---|---|---|---|
| C1 | 1 | RRARSVAS | Figure 8 |
| C2 | 2 | SSARSVAS | Figure 8 |
| Z1 | 3 | GTAMTAGA | Figure 8 |
| Z2 | 4 | GTAMGAGA | Figure 8 |
| Z3 | 5 | RTAMTAGA | Figure 9 |
| Z4 | 6 | RTAMGAGA | Figure 9 |
| Z5 | 7 | RTAMSAGA | Figure 9 |
| Z6 | 8 | RTAGSAGA | Figure 9 |
| | | | |
| C1S | 9 | Ac-K(Dabcyl)-RRARSVAS-E(Edans) | Figure 10 |
| Z3S | 10 | Ac-EG-K(Dabcyl)-RTAMTAGA-E(Edans)-GE | Figure 10 |
| | | | |
| C1Sc1 | 11 | K-(Dabcyl)-RRAR | Figure 10 |
| C1Sc2 | 12 | SVAS-E(Edans) | Figure 10 |

**Supplementary Table 2. Cleavage of proposed PSLs via furin within 15 min (Figure 3B).**

| Abbreviation | Cleavage [%] | Standard deviation [%] |
|---|---|---|
| C1 | 77.07 | 2.06 |
| C2 | 4.62 | 2.21 |
| Z1 | -2.36 | 0.86 |
| Z2 | 3.43 | 1.15 |
| Z3 | 26.79 | 2.71 |
| Z4 | 3.39 | 1.58 |
| Z5 | 26.16 | 2.19 |
| Z6 | 7.61 | 1.08 |

**Supplementary Table 3. Relative occurrence of amino acids for trypsin digested proteome in %.**

| | P6 [%] | P5 [%] | P4 [%] | P3 [%] | P2[%] | P1 [%] | P1' [%] | P2' [%] | P3' [%] | P4' [%] | P5' [%] | P6' [%] |
|---|---|---|---|---|---|---|---|---|---|---|---|---|
| A | 2.7 | 0.9 | 2.7 | 4.8 | 7.8 | 13.3 | 12.0 | 12.0 | 9.2 | 10.6 | 8.1 | 10.6 |
| C | 0.5 | 0.4 | 0.5 | 0.5 | 0.2 | 1.1 | 0.2 | 0.5 | 0.7 | 1.6 | 1.2 | 0.5 |
| D | 1.2 | 1.1 | 1.1 | 1.2 | 1.1 | 1.4 | 3.4 | 3.2 | 5.3 | 4.8 | 4.8 | 6.2 |
| E | 1.1 | 0.9 | 1.2 | 1.1 | 0.9 | 0.5 | 2.8 | 4.6 | 6.9 | 6.0 | 6.4 | 4.8 |
| F | 1.4 | 1.2 | 1.1 | 1.8 | 2.8 | 10.6 | 0.5 | 2.5 | 4.6 | 4.2 | 1.9 | 2.8 |
| G | 2.7 | 3.9 | 4.4 | 0.9 | 4.1 | 3.2 | 11.8 | 8.8 | 7.2 | 9.0 | 6.5 | 7.6 |
| H | 0.4 | 0.7 | 0.2 | 1.8 | 0.9 | 3.4 | 10.1 | 3.7 | 5.1 | 4.2 | 4.2 | 4.6 |
| I | 1.6 | 1.2 | 2.5 | 3.0 | 2.3 | 3.7 | 7.2 | 7.6 | 6.7 | 8.5 | 7.6 | 5.8 |
| K | 0.0 | 0.0 | 0.0 | 0.0 | 0.2 | 0.0 | 1.1 | 1.2 | 0.5 | 0.2 | 0.7 | 2.1 |
| L | 1.4 | 3.0 | 1.8 | 2.8 | 6.9 | 11.0 | 3.9 | 11.3 | 10.1 | 9.2 | 10.2 | 10.6 |
| M | 1.1 | 1.2 | 1.9 | 1.2 | 3.5 | 18.6 | 0.9 | 1.6 | 1.4 | 0.5 | 2.1 | 1.4 |
| N | 0.5 | 1.4 | 1.1 | 1.9 | 1.6 | 3.4 | 4.1 | 3.7 | 4.4 | 4.2 | 5.1 | 4.2 |
| P | 2.5 | 1.2 | 1.8 | 0.9 | 3.0 | 1.2 | 0.5 | 10.6 | 8.3 | 7.2 | 9.9 | 8.5 |
| Q | 0.4 | 1.1 | 1.4 | 2.1 | 2.8 | 1.4 | 3.5 | 4.6 | 3.9 | 5.3 | 4.6 | 4.9 |
| R | 0.2 | 0.2 | 0.2 | 0.2 | 0.0 | 0.0 | 2.5 | 1.4 | 1.8 | 1.8 | 1.9 | 5.3 |
| S | 1.8 | 0.9 | 2.7 | 2.3 | 4.1 | 3.2 | 12.4 | 3.7 | 5.1 | 4.1 | 4.9 | 4.1 |
| T | 0.7 | 1.4 | 2.3 | 4.8 | 6.4 | 6.2 | 13.8 | 5.1 | 6.7 | 6.7 | 6.4 | 6.5 |
| V | 1.4 | 3.4 | 1.6 | 4.6 | 3.2 | 5.5 | 6.4 | 11.8 | 9.4 | 9.5 | 8.5 | 6.4 |
| W | 0.2 | 0.2 | 0.4 | 0.5 | 0.4 | 0.9 | 0.2 | 0.7 | 0.5 | 0.2 | 0.9 | 0.0 |
| Y | 0.7 | 0.4 | 1.4 | 1.6 | 2.3 | 11.3 | 2.8 | 1.2 | 2.1 | 2.1 | 3.9 | 1.9 |

**Supplementary Table 4. Relative occurrence of amino acids for GluC digested proteome in %.**

| | P6 [%] | P5 [%] | P4 [%] | P3 [%] | P2 [%] | P1 [%] | P1' [%] | P2' [%] | P3' [%] | P4'[%] | P5' [%] | P6' [%] |
|---|---|---|---|---|---|---|---|---|---|---|---|---|
| A | 3.6 | 5.5 | 7.3 | 10.3 | 12.7 | 8.5 | 9.7 | 15.8 | 14.5 | 7.9 | 11.5 | 11.5 |
| C | 0.0 | 0.6 | 0.6 | 0.0 | 1.2 | 1.2 | 0.0 | 1.2 | 0.6 | 0.0 | 0.6 | 0.0 |
| D | 1.2 | 4.8 | 4.8 | 3.0 | 3.6 | 0.6 | 1.8 | 1.2 | 4.8 | 3.6 | 3.6 | 4.8 |
| E | 0.0 | 0.0 | 0.0 | 0.0 | 0.0 | 0.0 | 0.0 | 1.8 | 4.8 | 6.1 | 3.0 | 5.5 |
| F | 1.2 | 0.6 | 3.0 | 4.8 | 4.2 | 7.3 | 0.0 | 1.2 | 1.2 | 6.7 | 2.4 | 2.4 |
| G | 4.2 | 2.4 | 3.6 | 4.2 | 3.6 | 3.6 | 9.7 | 10.3 | 11.5 | 10.3 | 10.3 | 10.9 |
| H | 1.2 | 1.2 | 0.6 | 1.2 | 0.6 | 3.0 | 3.6 | 1.8 | 4.2 | 4.8 | 1.8 | 0.6 |
| I | 3.6 | 3.6 | 3.6 | 6.7 | 4.2 | 3.0 | 1.8 | 4.2 | 5.5 | 4.8 | 9.7 | 6.1 |
| K | 4.8 | 6.7 | 8.5 | 7.3 | 9.1 | 12.7 | 4.8 | 10.3 | 10.9 | 10.9 | 4.8 | 8.5 |
| L | 6.7 | 4.8 | 6.1 | 8.5 | 11.5 | 10.3 | 1.2 | 4.8 | 2.4 | 6.7 | 4.2 | 1.8 |
| M | 1.8 | 1.8 | 3.6 | 3.6 | 6.1 | 6.1 | 0.0 | 1.2 | 1.8 | 0.6 | 0.6 | 0.6 |
| N | 0.6 | 1.8 | 3.0 | 1.8 | 3.6 | 4.2 | 1.2 | 3.0 | 4.8 | 4.2 | 1.8 | 6.1 |
| P | 4.8 | 2.4 | 1.2 | 1.2 | 3.6 | 3.0 | 0.0 | 9.1 | 4.8 | 1.2 | 6.1 | 5.5 |
| Q | 3.6 | 1.2 | 2.4 | 3.0 | 1.2 | 4.8 | 4.2 | 3.6 | 3.6 | 3.0 | 4.8 | 1.2 |
| R | 7.9 | 12.7 | 7.9 | 13.9 | 12.7 | 21.8 | 49.1 | 7.9 | 8.5 | 8.5 | 10.9 | 6.1 |
| S | 3.6 | 3.6 | 4.8 | 4.8 | 5.5 | 3.0 | 6.7 | 4.8 | 5.5 | 7.9 | 8.5 | 7.9 |
| T | 1.8 | 2.4 | 5.5 | 3.0 | 2.4 | 0.6 | 0.6 | 6.1 | 4.2 | 3.0 | 4.8 | 6.7 |
| V | 2.4 | 7.9 | 5.5 | 8.5 | 6.1 | 1.2 | 4.2 | 10.9 | 4.2 | 7.9 | 8.5 | 10.3 |
| W | 0.6 | 1.2 | 1.8 | 1.2 | 0.6 | 0.6 | 0.0 | 0.0 | 0.0 | 0.0 | 0.6 | 0.0 |
| Y | 1.8 | 0.6 | 1.2 | 0.6 | 4.2 | 3.0 | 1.2 | 0.6 | 1.8 | 1.8 | 1.2 | 2.4 |

### LIST OF REFERENCES

1. Pulte, D. and H. Brenner, Changes in survival in head and neck cancers in the late 20th and early 21st century: a period analysis. The oncologist, 2010. 15(9): p. 994-1001.
2. Sung, H., et al., Global Cancer Statistics 2020: GLOBOCAN Estimates of Incidence and Mortality Worldwide for 36 Cancers in 185 Countries. CA: A Cancer Journal for Clinicians, 2021. 71(3): p. 209-249.
3. Argiris, A., et al., Head and neck cancer. The Lancet, 2008. 371(9625): p. 1695-1709.
4. Pai, S.I. and W.H. Westra, Molecular Pathology of Head and Neck Cancer: Implications for Diagnosis, Prognosis, and Treatment. Annual Review of Pathology: Mechanisms of Disease, 2009. 4(1): p. 49-70.
5. Chow, L.Q.M., Head and Neck Cancer. New England Journal of Medicine, 2020. 382(1): p. 60-72.
6. Graboyes, E.M., et al., Association of Treatment Delays With Survival for Patients With Head and Neck Cancer: A Systematic Review. JAMA Otolaryngology-Head & Neck Surgery, 2019. 145(2): p. 166-177.
7. Amin, M.B., et al., The Eighth Edition AJCC Cancer Staging Manual: Continuing to build a bridge from a population-based to a more "personalized" approach to cancer staging. CA: A Cancer Journal for Clinicians, 2017. 67(2): p. 93-99.
8. Cramer, J.D., et al., The changing therapeutic landscape of head and neck cancer. Nature Reviews Clinical Oncology, 2019. 16(11): p. 669-683.
9. Johnson, D.E., et al., Head and neck squamous cell carcinoma. Nature Reviews Disease Primers, 2020. 6(1): p. 92.
10. Cognetti, D.M., R.S. Weber, and S.Y. Lai, Head and neck cancer. Cancer, 2008. 113(S7): p. 1911-1932.
11. Thomas, G., Furin at the cutting edge: From protein traffic to embryogenesis and disease. Nature Reviews Molecular Cell Biology, 2002. 3(10): p. 753-766.
12. Xia, S., et al., The role of furin cleavage site in SARS-CoV-2 spike protein-mediated membrane fusion in the presence or absence of trypsin. Signal Transduction and Targeted Therapy, 2020. 5(1): p. 92.
13. Hosaka, M., et al., ArgX-Lys/Arg-Arg motif as a signal for precursor cleavage catalyzed by furin within the constitutive secretory pathway. Journal of Biological Chemistry, 1991. 266(19): p. 12127-12130.
14. Izidoro, M.A., et al., A study of human furin specificity using synthetic peptides derived from natural substrates, and effects of potassium ions. Archives of Biochemistry and Biophysics, 2009. 487(2): p. 105-114.
15. Izaguirre, G., The Proteolytic Regulation of Virus Cell Entry by Furin and Other Proprotein Convertases. Viruses, 2019. 11(9).
16. Braun, E. and D. Sauter, Furin-mediated protein processing in infectious diseases and cancer. Clinical & Translational Immunology, 2019. 8(8): p. e1073.
17. Bassi, D.E., et al., Elevated furin expression in aggressive human head and neck tumors and tumor cell lines. Molecular Carcinogenesis, 2001. 31(4): p. 224-232.
18. Bassi, D.E., et al., Increased Furin Activity Enhances the Malignant Phenotype of Human Head and Neck Cancer Cells. The American Journal of Pathology, 2003. 162(2): p. 439-447.
19. Zhou, B. and S. Gao, Pan-Cancer Analysis of FURIN as a Potential Prognostic and Immunological Biomarker. Frontiers in molecular biosciences, 2021. 8: p. 648402-648402.
20. Shah, N., E.A. Osea, and G.J. Martinez, Accuracy of noninvasive hemoglobin and invasive point-of-care hemoglobin testing compared with a laboratory analyzer. International Journal of Laboratory Hematology, 2014. 36(1): p. 56-61.
21. Miesler, T., et al., Frugal Innovation for Point-of-Care Diagnostics Controlling Outbreaks and Epidemics. ACS Biomaterials Science & Engineering, 2020. 6(5): p. 2709-2725.
22. Shandilya, R., et al., Nanobiosensors: Point-of-care approaches for cancer diagnostics. Biosensors and Bioelectronics, 2019. 130: p. 147-165.
23. Schilling, O., U. auf dem Keller, and C.M. Overall, Protease Specificity Profiling by Tandem Mass Spectrometry Using Proteome-Derived Peptide Libraries, in Gel-Free Proteomics: Methods and Protocols, K. Gevaert and J. Vandekerckhove, Editors. 2011, Humana Press: Totowa, NJ. p. 257-272.
24. Schilling, O., U. auf dem Keller, and C.M. Overall, Factor Xa subsite mapping by proteome-derived peptide libraries improved using WebPICS, a resource for proteomic identification of cleavage sites. Biol Chem, 2011. 392(11): p. 1031-7.
25. Rawlings, N.D., et al., The MEROPS database of proteolytic enzymes, their substrates and inhibitors in 2017 and a comparison with peptidases in the PANTHER database. Nucleic Acids Research, 2018. 46(D1): p. D624-D632.
26. Zhang, Y., et al., A second functional furin site in the SARS-CoV-2 spike protein. Emerging Microbes & Infections, 2022. 11(1): p. 182-194.
27. Tian, S., et al., FurinDB: A Database of 20-Residue Furin Cleavage Site Motifs, Substrates and Their Associated Drugs. International Journal of Molecular Sciences, 2011. 12(2).
28. Basak, A., et al., Implication of the proprotein convertases furin, PC5 and PC7 in the cleavage of surface glycoproteins of Hong Kong, Ebola and respiratory syncytial viruses: a comparative analysis with fluorogenic peptides. Biochemical Journal, 2001. 353(3): p. 537-545.

## Claims

1. A diagnostic sensor comprising:
a quenching moiety R1,
a Furin-sensitive peptide Pep, and
a signaling moiety R2,
wherein the quenching moiety R1 suppresses the signal from the signaling moiety R2 in the said sensor.

2. The sensor of claim 1,
A) wherein the Furin-sensitive peptide Pep comprises amino acid residues having the sequence P4-P3-P2-P1-P1'-P2'-P3'-P4' (SEQ ID NO: 17), wherein
P4 is independently selected from G and R, preferably wherein P4 is R
P3 is T,
P2 is A,
P1 is independently selected from M and G, preferably wherein P1 is M,
P1' is independently selected from T, G and S,
P2' is A,
P3' is G, and
P4' is A;
or
B) wherein the Furin-sensitive peptide Pep comprises amino acid residues having the sequence RRARSVAS (SEQ ID NO: 1) or SSARSVAS (SEQ ID NO: 2), particularly RRARSVAS.

3. The sensor of claim 1 or 2, further comprising,
a first additional moiety L1,
a second additional moiety L2,
particularly wherein the sensor has a structure selected from the group consisting of R1-L1-Pep-L2-R2 , L1-R1-Pep-L2-R2, L1-R1-Pep-R2-L2 and R1-L2-Pep-R2-L2.

4. The sensor of any one of claims 1 to 3, wherein the quenching moiety R1 comprises a moiety selected from the group consisting of commercially available quenching moieties,
particularly wherein R1 comprises optionally modified 4-((4-(Dimethylamino)phenyl)azo)benzoic acid, especially wherein R1 is acetylated, in particular wherein R1 comprises Ac-K(Dabcyl).

5. The sensor of any one of claims 1 to 4, wherein the fluorophore R2 comprises a moiety selected from the group consisting of small organic dyes, fluorescent proteins, and quantum dots,
particularly wherein R2 comprises 5-((2-Aminoethyl)amino)naphthalene-1-sulfonic acid, especially wherein R2 comprises -E(Edans).

6. The sensor of any one of claims 3 to 5, wherein
A) L1 comprises a moiety selected from the group consisting of linkers, natural polymers and synthetic polymers,
and/or
B) L2 comprises a moiety selected from the group consisting of linkers, natural polymers and synthetic polymers.

7. The sensor of any one of claims 1 to 6 for use in medicine,
particularly for use in a method of diagnosing cancer.

8. The sensor for use of claim 7, wherein the method comprises determining furin activity in a subject, particularly in the oral cavity of the subject,
particularly wherein an increased level of furin activity relative to a control level is indicative of the subject having cancer.

9. The sensor for use of any one of claims 7 and 8, wherein the method is a method of diagnosing a subject for cancer, and wherein the method comprises:
a) determining a level of furin activity in the subject,
b) optionally comparing the level of furin activity to a control level of furin activity,
c) diagnosing whether the subject has cancer, wherein an increased level of furin activity is indicative of the subject having cancer.

10. The sensor for use of any one of claims 7 to 9,
A) wherein the subject is a mammalian subject, particularly a human subject;
and/or
B) wherein the cancer is a head and neck cancer, particularly wherein the cancer is HNSCC.

11. A furin-sensitive peptide having a length of from 8 to 16 amino acids, wherein
A) the Furin-sensitive peptide Pep comprises amino acid residues having the sequence P4-P3-P2-P1-P1'-P2'-P3'-P4' (SEQ ID NO: 17), wherein
P4 is independently selected from G and R, preferably wherein P4 is R
P3 is T,
P2 is A,
P1 is independently selected from M and G, preferably wherein P1 is M,
P1' is independently selected from T, G and S,
P2' is A,
P3' is G, and
P4' is A;
or
B) the Furin-sensitive peptide Pep comprises amino acid residues having the sequence RRARSVAS (SEQ ID NO: 1) or SSARSVAS (SEQ ID NO: 2), particularly RRARSVAS.

12. The furin-sensitive peptide according to claim 11 for use in medicine, particularly wherein the use is further defined as in any one of claims 7 to 10.

13. A diagnostic kit comprising the sensor in accordance with any one of claims 1 to 6 and/or the peptide in accordance with claim 11.

14. A method of determining furin activity in a sample, wherein the method comprises using a sensor in accordance with any one of claims 1 to 6, a peptide in accordance with claim 11, or a kit in accordance with claim 13.

15. The method of claim 14, wherein
A) the sample has been obtained from a subject, particularly from the oral cavity of a subject,
and/or
B) the method is a method of diagnosing cancer, in particular wherein an increased level of furin activity relative to the level of a control is indicative of the subject having cancer;
especially wherein the method comprises
a) determining a level of furin activity in a sample obtained from the subject,
b) optionally comparing the level in the said sample to a control level of furin activity,
c) diagnosing whether the subject has cancer, wherein an increased level of furin activity is indicative of the subject having cancer.
